# EUROPEAN PATENT SPECIFICATION

(11) **EP 3 257 511 B1**
(45) Date of publication and mention of the grant of the patent: **24.10.2018**
(21) Application number: 15882014.2
(22) Date of filing: 10.09.2015
(51) Int. Cl.: A61K 31/46, A61K 9/20, A61K 47/04, A61K 47/12, A61K 47/14, A61K 47/20, A61K 47/32, A61P 1/06, A61P 13/06, A61P 21/02, A61P 25/08

(54) **ORALLY DISINTEGRATING TABLET AND METHOD FOR MANUFACTURING SAME**
IM MUND ZERFALLENDE TABLETTE UND VERFAHREN ZUR HERSTELLUNG DAVON
COMPRIMÉ À DÉSINTÉGRATION INTRABUCCALE ET SON PROCÉDÉ DE FABRICATION

(30) Priority: 10.02.2015 JP 2015024699; 14.07.2015 JP 2015140608
(43) Date of publication of application: 20.12.2017
(73) Proprietor: FUJIFILM Corporation, Tokyo 106-8620 (JP)
(72) Inventor: OKADA, Kotaro, Ashigarakami-gun Kanagawa 258-8577 (JP); MORI, Hisahiro, Ashigarakami-gun Kanagawa 258-8577 (JP); TSUJIHATA, Shigetomo, Ashigarakami-gun Kanagawa 258-8577 (JP); SAKATA, Yoshinori, Ashigarakami-gun Kanagawa 258-8577 (JP)
(74) Representative: Hoffmann Eitle
(86) International application number: PCT/JP2015/075749
(87) International publication number: WO 2016/129140

(56) References cited:
- WO-A1-2014/081338
- JP-A- S5 176 413
- JP-A- 2000 119 198
- JP-A- 2006 519 202
- JP-A- 2011 513 204
- JP-A- 2012 036 140
- JP-A- 2012 056 909

## Description

### BACKGROUND OF THE INVENTION

### 1. Field of the Invention

The present invention relates to an orally disintegrating tablet and a method for producing the same.

### 2. Description of the Related Art

In recent years, there is a demand for the development of orally disintegrating tablets that can be taken in without water, as preparations that can be more easily taken in, for the purpose of enhancing the compliance of highly aged patients, patients with difficulties in swallowing, patients with restricted water intake, and the like.

An orally disintegrating tablet is a formulation with which the bitter taste of a drug can be easily felt because the tablet is rapidly disintegrated in the oral cavity, unlike other ordinary tablets.

Butylscopolamine bromide, an antispasmodic agent, is a drug having a bitter taste, and therefore, in a case in which the drug is produced into a dosage form of an orally disintegrating tablet, it is necessary to conceal (mask) the bitter taste so that the bitter taste of butylscopolamine bromide cannot be felt.

Regarding a method for concealing the bitter taste of a drug in an orally disintegrating tablet, a physical masking method of coating the drug with a film including a polymer and the like is known.

For example, WO2005/105045A discloses a time-limited type particulate pharmaceutical composition for oral administration, which has a core particle containing a drug at the center; a layer containing two kinds of water-soluble components such as an insolubilization accelerator and an insolubilizing substance as an intermediate layer; and a water infiltration amount-controlling layer for controlling the rate of water infiltration into the interior as an outermost layer.

In the pharmaceutical composition disclosed in WO2005/105045A, a drug having an unpleasant taste such as a bitter taste is coated with an intermediate layer containing two kinds of water-soluble components such as an insolubilization accelerator and an insolubilizing substance, and solubility of the intermediate layer is controlled by further coating the intermediate layer that is covering the drug, with a water infiltration amount controlling layer as an outermost layer containing a water-insoluble substance. Thus, dissolution of the drug having an unpleasant taste is controlled.

### SUMMARY OF THE INVENTION

Generally, an orally disintegrating tablet is configured to include a fine granule containing a drug, and excipient components on the outside of the fine granule.

However, it has been clearly known that when it is attempted to coat butylscopolamine bromide, which is a drug having a bitter taste, with the layers disclosed in WO2005/105045A, aggregation of particles including butylscopolamine bromide occurs, and it is difficult to produce fine granules.

The present invention is made in view of such circumstances as described above, and an object according to an embodiment of the invention is to provide an orally disintegrating tablet that conceals a bitter taste attributed to butylscopolamine bromide.

Furthermore, an object according to another embodiment of the invention is to provide a method for producing an orally disintegrating tablet, by which an orally disintegrating tablet that conceals a bitter taste attributed to butylscopolamine bromide can be produced highly efficiently.

Specific means for attaining the objects includes the following aspects.
[1] An orally disintegrating tablet comprising: fine granules, each fine granule having, at its center, an active pharmaceutical ingredient-containing core that comprises butylscopolamine bromide and water-insoluble particles, and having an intermediate layer that comprises water-insoluble particles and coats the active pharmaceutical ingredient-containing core, and a bitterness-masking layer that comprises talc and at least one water-insoluble polymer, in sequence from the active pharmaceutical ingredient-containing core side; and an excipient component positioned on the outside of the fine granules.
[2] The orally disintegrating tablet according to [1], wherein the fine granule further has an overcoat layer comprising an anionic substance having a pKa of 3.5 or lower, as a layer positioned on an outer side of the bitterness-masking layer.
[3] The orally disintegrating tablet according to [1] or [2], wherein the excipient component on the outside of the fine granules comprises an anionic substance having a pKa of 3.5 or lower.
[4] The orally disintegrating tablet according to [2] or [3], wherein the anionic substance having a pKa of 3.5 or lower is sodium lauryl sulfate.
[5] The orally disintegrating tablet according to any one of [1] to [4], wherein a thickness of the intermediate layer is 10 µm or less.
[6] The orally disintegrating tablet according to any one of [1] to [5], wherein the water-insoluble polymer comprises at least one selected from the group consisting of an aminoalkyl methacrylate copolymer RS and an ethyl acrylate/methyl methacrylate copolymer.
[7] The orally disintegrating tablet according to any one of [1] to [6], wherein the bitterness-masking layer comprises triethyl citrate at a proportion of from 5% by mass to 15% by mass with respect to a total solid content of the water-insoluble polymer.
[8] The orally disintegrating tablet according to any one of [1] to [7], wherein the water-insoluble particles included in the active pharmaceutical ingredient-containing core and the intermediate layer are formed of at least one selected from the group consisting of hydrous silicon dioxide, light anhydrous silicic acid, sodium stearyl fumarate, magnesium stearate, and talc.
[9] The orally disintegrating tablet according to any one of [1] to [8], wherein an average particle size of the fine granules is from 100 µm to 500 µm.
[10] The orally disintegrating tablet according to any one of [1] to [9], wherein the intermediate layer has an underlying layer and an overlying layer in sequence from the active pharmaceutical ingredient-containing core side.
[11] The orally disintegrating tablet according to [10], wherein the underlying layer comprises water-insoluble particles, or water-insoluble particles and a coating film component, and a ratio of a content of the water-insoluble particles to a content of the coating film component in the underlying layer is from 1.0:0.0 to 1.0:1.0 on a mass basis.
[12] The orally disintegrating tablet according to any one of [1] to [11], wherein the active pharmaceutical ingredient-containing core has a core particle at its center, and has an active pharmaceutical ingredient layer comprising butylscopolamine bromide and water-insoluble particles on an outside of the core particle.
[13] A method for producing an orally disintegrating tablet, the method comprising: a step of obtaining fine granules by a production process comprising: (A) spraying, onto a core particle that is configured to serve as a center of an active pharmaceutical ingredient-containing core, a spray liquid that comprises butylscopolamine bromide and water-insoluble particles and that is for forming an active pharmaceutical ingredient layer, so as to coat the core particle with the active pharmaceutical ingredient layer; (B) spraying, onto the active pharmaceutical ingredient-containing core in which the core particle has been coated with the active pharmaceutical ingredient layer, a spray liquid that comprises water-insoluble particles and that is for forming an intermediate layer, so as to coat the active pharmaceutical ingredient-containing core with the intermediate layer; and (C) spraying, onto the active pharmaceutical ingredient-containing core that has been coated with at least the intermediate layer, a spray liquid that comprises talc and at least one water-insoluble polymer and that is for forming a bitterness-masking layer, so as to coat the active pharmaceutical ingredient-containing core with the bitterness-masking layer; and mixing the obtained fine granules with an excipient component.
[14] The method for producing an orally disintegrating tablet according to [13], wherein the water-insoluble polymer comprises at least one selected from the group consisting of an aminoalkyl methacrylate copolymer RS and an ethyl acrylate/methyl methacrylate copolymer.
[15] The method for producing an orally disintegrating tablet according to [13] or [14], wherein the water-insoluble particles included in the spray liquid for forming an active pharmaceutical ingredient layer and the spray liquid for forming an intermediate layer are formed of at least one selected from the group consisting of hydrous silicon dioxide, light anhydrous silicic acid, sodium stearyl fumarate, magnesium stearate, and talc.
[16] The method for producing an orally disintegrating tablet according to any one of [13] to [15], wherein the spray liquid for forming a bitterness-masking layer comprises triethyl citrate at a proportion of from 5% by mass to 15% by mass with respect to a total solid content of the water-insoluble polymer.
[17] The method for producing an orally disintegrating tablet according to any one of [13] to [16], wherein the (B) comprises: S(B1) spraying, onto the active pharmaceutical ingredient-containing core, a spray liquid that comprises water-insoluble particles and that is for forming an underlying layer, so as to coat the active pharmaceutical ingredient-containing core with the underlying layer; and (B2) spraying, onto the active pharmaceutical ingredient-containing core that has been coated with at least the underlying layer, a spray liquid that comprises water-insoluble particles and that is for forming an overlying layer, so as to coat the active pharmaceutical ingredient-containing core with the overlying layer.
[18] The method for producing an orally disintegrating tablet according to [17], wherein a content of the water-insoluble particles in the spray liquid for forming an underlying layer is from 1.0% by mass to 50.0% by mass with respect to a total amount of the spray liquid for forming an underlying layer.
[19] The method for producing an orally disintegrating tablet according to any one of [13] to [18], wherein the water-insoluble particles included in the spray liquid for forming an active pharmaceutical ingredient layer and the spray liquid for forming an intermediate layer are formed of at least one selected from the group consisting of hydrous silicon dioxide, light anhydrous silicic acid, sodium stearyl fumarate, and talc.
[20] The method for producing an orally disintegrating tablet according to any one of [13] to [19], wherein the excipient component comprises an anionic substance having a pKa of 3.5 or lower.

According to the embodiment of the invention, an orally disintegrating tablet that conceals the bitter taste attributed to butylscopolamine bromide can be provided.

Furthermore, according to the other embodiment of the invention, a method for producing an orally disintegrating tablet, by which an orally disintegrating tablet that conceals the bitter taste attributed to butylscopolamine bromide can be produced highly efficiently, can be provided.

### DESCRIPTION OF THE PREFERRED EMBODIMENTS

Hereinafter, specific embodiments of the invention will be described in detail; however, the invention is not intended to be limited by the following embodiments, and modifications can be applied as appropriate within the intended scope of the invention.

The numerical value range described using the expression of "from ... to ..." in the present specification means a range including the numerical values described before and after the symbol "to" as the minimum value and the maximum value, respectively.

According to the present specification, unless particularly stated otherwise, the amount of each component in the composition means, in a case in which a plurality of substances corresponding to the component exist in the composition, the total amount of the plurality of substances existing in the composition.

The term "average particle size" according to the present specification means the volume average particle size (Mv), and the value of the "average particle size" is a value measured using a laser diffraction scattering type particle size distribution analyzer. Regarding the analyzer, for example, LS 13 320 (product name) of Beckman Coulter, Inc. can be suitably used. However, the analyzer is not limited to this.

The term "water-insoluble particles" according to the present specification refers to particles having a solubility in water at 25°C of less than 0.1 g/L.

The term "layer" according to the present specification includes a configuration in which the entirety of an object of coating is coated, as well as a configuration in which a portion of an object of coating is coated.

According to the present specification, the term "step" includes not only an independent step, and even in a case in which a step is not clearly distinguishable from another step, as long as the desired purpose of the step is achieved, this step is included in the present term.

### [Orally disintegrating tablet]

The orally disintegrating tablet of the present disclosure is an orally disintegrating tablet including fine granules, each fine granule having, at the center, an active pharmaceutical ingredient-containing core that includes butylscopolamine bromide and water-insoluble particles, and having an intermediate layer that includes water-insoluble particles and covers the active pharmaceutical ingredient-containing core, and a bitterness-masking layer that includes talc and at least one water-insoluble polymer, in sequence from the active pharmaceutical ingredient-containing core side; and excipient components positioned on the outside of the fine granules.

The orally disintegrating tablet of the present disclosure may also include other components in addition to butylscopolamine bromide, water-insoluble particles, talc and a water-insoluble polymer as necessary, to the extent that the effects of the invention are not impaired.

In the following description, the various components of the orally disintegrating tablet of the present disclosure will be explained in detail.

### <<Fine granules>>

The orally disintegrating tablet of the present disclosure is an orally disintegrating tablet including fine granules, each fine granule having, at the center, an active pharmaceutical ingredient-containing core that includes butylscopolamine bromide and water-insoluble particles, and having an intermediate layer that includes water-insoluble particles and covers the active pharmaceutical ingredient-containing core, and a bitterness-masking layer that includes talc and at least one water-insoluble polymer, in sequence from the active pharmaceutical ingredient-containing core side. The fine granule according to the present disclosure may have another layer in addition to the intermediate layer and the bitterness-masking layer as necessary, to the extent that the effects of the invention are not impaired.

The phrase "having an active pharmaceutical ingredient-containing core at the center" is purported to indicate the positional relation between the active pharmaceutical ingredient-containing core and other layers such as the intermediate layer, and the phrase is not intended to imply that the active pharmaceutical ingredient-containing core is positioned at the center of the fine granule in a strict sense.

As a method for concealing the bitter taste of a drug in conventional orally disintegrating tablets, for example, a method of coating a drug having a bitter taste with the layers disclosed in WO2005/105045A is available.

However, when it is attempted to coat butylscopolamine bromide, which is a drug having a bitter taste, with the layers disclosed in WO2005/105045A, aggregation of the particles including butylscopolamine bromide occurs, and production of fine granules is made difficult.

The orally disintegrating tablet of the present disclosure can conceal a bitter taste attributed to butylscopolamine bromide, as each of the fine granules included in the orally disintegrating tablet (hereinafter, may be simply referred to as "fine granules") has, at the center, an active pharmaceutical ingredient-containing core that includes butylscopolamine bromide and water-insoluble particles, and has an intermediate layer that includes water-insoluble particles and covers the active pharmaceutical ingredient-containing core, and a bitterness-masking layer that includes talc and at least one water-insoluble polymer, in sequence from the active pharmaceutical ingredient-containing core side.

The reason why the orally disintegrating tablet of the present disclosure can conceal a bitter taste attributed to butylscopolamine bromide is speculated as follows.

In the orally disintegrating tablet of the present disclosure, since the active pharmaceutical ingredient-containing core that includes butylscopolamine bromide and water-insoluble particles is coated with an intermediate layer including water-insoluble particles, the bitter taste attributed to butylscopolamine bromide can be concealed. Also, in the orally disintegrating tablet of the present disclosure, since the fine granule has a bitterness-masking layer that includes talc and at least one water-insoluble polymer, on the outer side of the intermediate layer that covers the active pharmaceutical ingredient-containing core including butylscopolamine bromide, dissolution properties of the intermediate layer are controlled, and the bitter taste attributed to butylscopolamine bromide in the oral cavity can be concealed.

### <Active pharmaceutical ingredient-containing core>

The active pharmaceutical ingredient-containing core according to the present disclosure is an active pharmaceutical ingredient-containing core that includes butylscopolamine bromide and water-insoluble particles.

When the active pharmaceutical ingredient-containing core includes water-insoluble particles together with butylscopolamine bromide, satisfactory productivity is obtained.

### (Butylscopolamine bromide)

The active pharmaceutical ingredient-containing core includes butylscopolamine bromide as an active ingredient.

Butylscopolamine bromide is a drug which is known as an antispasmodic agent that suppresses excessive movement and convulsion of the muscles in internal organs, and has a bitter taste.

The content of butylscopolamine bromide in the active pharmaceutical ingredient-containing core is not particularly limited. Furthermore, the content of butylscopolamine bromide in the fine granules is also not particularly limited.

### (Water-insoluble particles)

The active pharmaceutical ingredient-containing core includes water-insoluble particles.

The water-insoluble particles are not particularly limited as long as they are formed from a pharmacologically acceptable component. The water-insoluble particles may be inorganic particles, may be organic particles, or may be inorganic/organic composite particles, as long as the particles are water-insoluble.

Specific examples of the material for the water-insoluble particles include lubricating agents such as hydrous silicon dioxide, light anhydrous silicic acid, sodium stearyl fumarate, magnesium stearate, calcium stearate, glycerin monostearate, and talc.

Among these, from the viewpoint of productivity, the material for the water-insoluble particles included in the active pharmaceutical ingredient-containing core is preferably at least one selected from the group consisting of hydrous silicon dioxide, light anhydrous silicic acid, sodium stearyl fumarate, magnesium stearate, and talc; more preferably at least one selected from the group consisting of hydrous silicon dioxide, light anhydrous silicic acid, sodium stearyl fumarate, and talc; and even more preferably at least one selected from the group consisting of hydrous silicon dioxide, light anhydrous silicic acid, and talc.

From the viewpoint of storage stability of the content of the active pharmaceutical ingredient, it is particularly preferable for the orally disintegrating tablet of the present disclosure that the active pharmaceutical ingredient-containing core includes hydrous silicon dioxide.

The active pharmaceutical ingredient-containing core may include one kind of water-insoluble particles alone, or two or more kinds of water-insoluble particles in combination.

The average particle size of the water-insoluble particles is not particularly limited.

The shape of the water-insoluble particles is not particularly limited, and the shape may be any of a spherical shape, an elliptical shape, a polygonal shape, a needle-like shape, an irregular shape, and the like.

The content of the water-insoluble particles in the active pharmaceutical ingredient-containing core is not particularly limited. For example, the content of the water-insoluble particles in the active pharmaceutical ingredient-containing core is preferably from 5 parts by mass to 50 parts by mass, more preferably from 10 parts by mass to 40 parts by mass, and even more preferably from 20 parts by mass to 30 parts by mass, with respect to 100 parts by mass of butylscopolamine bromide included in the active pharmaceutical ingredient-containing core, from the viewpoint of productivity.

### (Other components)

The active pharmaceutical ingredient-containing core may include other components in addition to butylscopolamine bromide and the water-insoluble particles as necessary, to the extent that the effects of the invention are not impaired.

Examples of the other components include an excipient, a disintegrant, and a binder. These other components can be appropriately selected according to the purpose. Furthermore, these other components may be components such that one component performs two or more functions.

### - Excipient -

An excipient contributes to an enhancement of formability of the active pharmaceutical ingredient-containing core.

The excipient is not particularly limited as long as it is a component that can function as an excipient and is a pharmacologically acceptable component, and any known excipient can be used.

Examples of the excipient include a saccharide, a sugar alcohol, a starch, crystalline cellulose, ethyl cellulose, anhydrous calcium phosphate, and magnesium aluminometasilicate.

Examples of the saccharide include lactose, sucrose, maltose, trehalose, and dextrin. Examples of the sugar alcohol include mannitol, erythritol, isomalt, lactitol, maltitol, sorbitol and xylitol. Examples of the starch include corn starch, potato starch, rice starch, and wheat starch.

### - Disintegrant -

A disintegrant can contribute to the acceleration of disintegrability of the active pharmaceutical ingredient-containing core.

The disintegrant is not particularly limited as long as it is a component that can function as a disintegrant and is a pharmacologically acceptable component, and any known disintegrant can be used.

Examples of the disintegrant include starches such as corn starch and potato starch; partially gelatinized starch, carboxymethyl starch sodium, carmellose, carmellose calcium, croscarmellose sodium, crospovidone, low-substituted hydroxypropyl cellulose, crystalline cellulose, hydroxypropyl starch, and sodium starch glycolate.

### - Binder -

A binder can contribute to an enhancement of formability of the active pharmaceutical ingredient-containing core.

The binder is not particularly limited as long as it is a component that can function as a binder and is a pharmacologically acceptable component, and any known binder can be used.

Examples of the binder include hydroxypropyl cellulose, hydroxypropyl methylcellulose, a carboxyvinyl polymer, carmellose sodium, polyethylene glycol, polyvinylpyrrolidone, polyvinyl alcohol, gum arabic, gelatin, gelatinized starch, and pullulan.

In a case in which the active pharmaceutical ingredient-containing core includes other components, the core may include one kind of other component, or may include two or more kinds of other components.

The content of the other components in the active pharmaceutical ingredient-containing core is not particularly limited, and the content can be appropriately set in accordance with the contents of butylscopolamine bromide and the water-insoluble particles.

### (Average particle size of active pharmaceutical ingredient-containing core)

The average particle size of the active pharmaceutical ingredient-containing core is not particularly limited, and for example, the average particle size is preferably from 100 µm to 400 µm, more preferably from 150 µm to 350 µm, and even more preferably from 200 µm to 300 µm.

When the average particle size of the active pharmaceutical ingredient-containing core is adjusted to be in the range described above, surface roughness at the time of taking the orally disintegrating tablet can be reduced, and therefore, deterioration of the feel of taking the orally disintegrating tablet can be avoided. Also, the active pharmaceutical ingredient-containing core can be produced satisfactorily.

### (Form of active pharmaceutical ingredient-containing core)

The active pharmaceutical ingredient-containing core is desirably a granulated material including butylscopolamine bromide and water-insoluble particles. The active pharmaceutical ingredient-containing core may be a granulated material in which butyl scopolamine bromide and water-insoluble particles are mixed with other components, or may be a granulated material in which the surface of core particles is coated with a layer including butylscopolamine bromide and water-insoluble particles (hereinafter, referred to as "active pharmaceutical ingredient layer" as appropriate).

It is preferable that the active pharmaceutical ingredient-containing core is in the form of a particle having a core particle at the center and having, on the outside of the core particle, an active pharmaceutical ingredient layer including butylscopolamine bromide and water-insoluble particles. Particularly, from the viewpoint of storage stability of the content of the active pharmaceutical ingredient, it is preferable that the orally disintegrating tablet includes hydrous silicon dioxide in the active pharmaceutical ingredient layer.

The phrase "having a core particle at the center" is purported to indicate the positional relation between the core particle and the active pharmaceutical ingredient layer, and the phrase is not intended to imply that the core particle is positioned at the center of the active pharmaceutical ingredient-containing core in a strict sense.

The active pharmaceutical ingredient-containing core may have an undercoat layer that includes other components, between the core particle and the active pharmaceutical ingredient layer.

### - Core particle -

A core particle is a particle that can serve as a base material when the active pharmaceutical ingredient-containing core is produced.

The component that forms the core particle is not particularly limited as long as it is a pharmacologically acceptable component. The component that forms the core particle may be a water-soluble particle, or may be a water-insoluble particle. It is preferable from the viewpoint of productivity that the component that forms the core particle is a water-insoluble particle.

The core particle may be a particle formed from a material such as mannitol, lactose, crystalline cellulose, magnesium aluminometasilicate, anhydrous calcium hydrogen phosphate, calcium silicate, magnesium oxide, or magnesium carbonate.

Among these, the core particle is more preferably a particle formed from crystalline cellulose, mannitol or the like.

For the core particle, a raw powder of the above-described component may be used per se, a granulated material of a raw powder of the above-described component may be used, or commercially available core particles may also be used.

Examples of commercially available core particles include FLORITE (trade name, calcium silicate, Eisai Food & Chemical Co., Ltd.), NONPAREIL (registered trademark) (mannitol, Freund Corporation), and CELPHERE (registered trademark) (crystalline cellulose, Asahi Kasei Chemicals Corporation).

Since the active pharmaceutical ingredient-containing core is produced by, for example, spraying a spray liquid including butylscopolamine bromide and water-insoluble particles (hereinafter, referred to as "spray liquid for an active pharmaceutical ingredient layer") onto core particles, from the viewpoint of facilitating spraying of the spray liquid for an active pharmaceutical ingredient layer onto core particles, the core particles are preferably core particles having a smooth surface. From the viewpoint of making the particle size distribution of the active pharmaceutical ingredient-containing particles uniform, the core particles are preferably core particles having a uniformized particle size distribution.

The average particle size of the core particles is not particularly limited. For example, the average particle size of the core particles is preferably from 50 µm to 350 µm, more preferably from 100 µm to 300 µm, and even more preferably from 150 µm to 250 µm.

When the average particle size of the core particles is adjusted to be in the range described above, it becomes easier to obtain an active pharmaceutical ingredient-containing core having a high degree of sphericity, and therefore, when the active pharmaceutical ingredient-containing core is coated with a layer such as an intermediate layer or a bitterness-masking layer, uniformity of coating can be enhanced.

The proportion occupied by the active pharmaceutical ingredient-containing core in a fine granule is not particularly limited. For example, the proportion occupied by the active pharmaceutical ingredient-containing core in a fine granule is preferably from 30% by mass to 70% by mass, more preferably from 40% by mass to 70% by mass, and even more preferably from 40% by mass to 60% by mass, with respect to the mass of the fine granule.

### <Intermediate layer>

The intermediate layer according to the present disclosure includes water-insoluble particles.

The intermediate layer is a layer that covers the active pharmaceutical ingredient-containing core, and is positioned between the active pharmaceutical ingredient-containing core and the bitterness-masking layer.

In the orally disintegrating tablet of the present disclosure, since the fine granules have an intermediate layer, the bitter taste attributed to butylscopolamine bromide can be concealed. Furthermore, in the orally disintegrating tablet of the present disclosure, since the intermediate layer, which covers the active pharmaceutical ingredient-containing core including butylscopolamine bromide, includes water-insoluble particles, satisfactory productivity is obtained.

It is desirable that the intermediate layer is in a state of covering at least a portion of the surface of the active pharmaceutical ingredient-containing core. It is preferable that the intermediate layer covers 1/4 or more, and more preferably 1/2 or more, of the surface of the active pharmaceutical ingredient-containing core. From the viewpoint that the effects of the invention can be provided more noticeably, it is most preferable that the intermediate layer covers the entire surface of the active pharmaceutical ingredient-containing core.

### (Water-insoluble particles)

Water-insoluble particles are not particularly limited as long as the particles are formed from a pharmacologically acceptable component. The water-insoluble particles may be inorganic particles, may be organic particles, or may be inorganic/organic composite particles, as long as the particles are particles that are water-insoluble.

Specific examples of the material of the water-insoluble particles include lubricating agents such as hydrous silicon dioxide, light anhydrous silicic acid, sodium stearyl fumarate, magnesium stearate, calcium stearate, glycerin monostearate, and talc.

Among these, from the viewpoint of productivity, the material that forms the water-insoluble particles that are included in the intermediate layer is preferably at least one selected from the group consisting of hydrous silicon dioxide, light anhydrous silicic acid, sodium stearyl fumarate, magnesium stearate, and talc; more preferably at least one selected from the group consisting of hydrous silicon dioxide, light anhydrous silicic acid, sodium stearyl fumarate, and talc; and even more preferably at least one selected from the group consisting of hydrous silicon dioxide, light anhydrous silicic acid, and talc.

It is particularly preferable that the orally disintegrating tablet of the present disclosure includes hydrous silicon dioxide in the intermediate layer, from the viewpoint of storage stability of the content of the active pharmaceutical ingredient.

The intermediate layer may include one kind of the water-insoluble particles alone, or two or more kinds thereof in combination.

The average particle size of the water-insoluble particles is not particularly limited.

The shape of the water-insoluble particles is not particularly limited, and the shape may be any of a spherical shape, an elliptical shape, a polygonal shape, a needle-like shape, an irregular shape and the like.

The content of the water-insoluble particles in the intermediate layer is not particularly limited. For example, the content of the water-insoluble particles in the intermediate layer is preferably from 1 part by mass to 30 parts by mass, more preferably from 3 parts by mass to 25 parts by mass, and even more preferably from 5 parts by mass to 20 parts by mass, with respect to 100 parts by mass of butylscopolamine bromide included in the active pharmaceutical ingredient-containing core, from the viewpoint of productivity.

### (Coating film component)

The intermediate layer may include a coating film component.

The coating film component in the intermediate layer is not particularly limited as long as it is a pharmacologically acceptable component, and examples thereof include a water-soluble polymer and a water-insoluble polymer.

According to the present specification, the "water-insoluble polymer" as the coating film component that is included in the intermediate layer, means a polymer having a solubility in water at 20°C of less than 10 g/L.

Examples of the water-soluble polymer include a water-soluble cellulose derivative, a water-soluble vinyl polymer derivative, a water-soluble acrylic acid copolymer, and a polyhydric alcohol polymer.

Examples of the water-insoluble polymer include a water-insoluble cellulose ether and a water-insoluble acrylic acid copolymer.

The coating film component is preferably a water-soluble polymer from the viewpoint of productivity, and at least one water-soluble polymer selected from the group consisting of a water-soluble cellulose derivative and a water-soluble vinyl polymer derivative is preferred, while a water-soluble cellulose derivative is more preferred.

The water-soluble cellulose derivative is not particularly limited, and examples thereof include hydroxypropyl cellulose (HPC), hydroxypropyl methylcellulose (HPMC), methyl cellulose (MC), and carboxymethyl ethylcellulose (CMEC).

Among these, the water-soluble cellulose derivative is preferably at least one selected from hydroxypropyl cellulose (HPC) and hydroxypropyl methylcellulose (HPMC), and more preferably hydroxypropyl methylcellulose (HPMC).

In a case in which the intermediate layer includes a water-soluble cellulose derivative, the intermediate layer may include one kind of a water-soluble cellulose derivative, or may include two or more kinds thereof.

In a case in which the intermediate layer includes a water-soluble cellulose derivative, the content of the cellulose derivative in the intermediate layer is not particularly limited. For example, from the viewpoint of productivity, the content of the cellulose derivative in the intermediate layer is preferably from 10% by mass to 50% by mass, more preferably from 15% by mass to 45% by mass, and even more preferably from 25% by mass to 35% by mass, with respect to the total mass of the component that forms the intermediate layer.

### (Other components)

The intermediate layer may include other components as necessary, in addition to the water-insoluble particles, and a film coating component, which is an optional component, to the extent that the effects of the invention are not impaired.

Examples of the other components include an excipient, a disintegrant, and a binder. These other components can be appropriately selected according to the purpose.

Since the other components in the intermediate layer have the same meaning as the other components explained in the section concerning the active pharmaceutical ingredient-containing core, further explanation will not be provided here.

In a case in which the intermediate layer includes other components, the intermediate layer may include one kind of the other components, or may include two or more kinds thereof.

The content of the other components in the intermediate layer is not particularly limited, and the content can be appropriately set in accordance with the contents of the water-insoluble particles, and the coating film component, which is an optional component.

### (Configuration of intermediate layer)

The intermediate layer may be a single layer, or may be a multilayer of two or more layers.

The intermediate layer may have an underlying layer and an overlying layer in sequence from the active pharmaceutical ingredient-containing core side. That is, the intermediate layer may be formed into, for example, three or more layers of an underlying layer, one or more interlying layers, and an overlying layer, in sequence from the active pharmaceutical ingredient-containing core side, or may be formed into two layers of an underlying layer and an overlying layer.

The term "underlying layer" according to the present specification refers to a layer that is positioned most closely to the active pharmaceutical ingredient-containing core side among the layers that form the intermediate layer, the term "overlying layer" refers to a layer that is positioned most closely to the bitterness-masking layer side among the layers that form the intermediate layer, and the term "interlying layer" refers to a layer that is positioned between the underlying layer and the overlying layer. The underlying layer, the overlying layer, and the interlying layer differ from each other in the type, content, content ratio and the like of the components that form the respective layers.

In a case in which the intermediate layer has an underlying layer, it is preferable that the water-insoluble particles, or water-insoluble particles and a coating film component are included in the underlying layer. The ratio between the content of the water-insoluble particles and the content of the coating film component in the underlying layer is preferably from 1.0:0.0 to 1.0:1.0 on a mass basis.

When the intermediate layer has an underlying layer including water-insoluble particles, or water-insoluble particles and a coating film component, and the ratio between the content of the water-insoluble particles and the content of the coating film component in the underlying layer is adjusted to be in the range described above, productivity can be further enhanced.

In a case in which the intermediate layer has an underlying layer, from the viewpoint of productivity, the material of the water-insoluble particles included in the underlying layer is preferably a lubricating agent such as hydrous silicon dioxide, light anhydrous silicic acid, sodium stearyl fumarate, magnesium stearate, calcium stearate, glycerin monostearate, or talc; more preferably at least one selected from the group consisting of hydrous silicon dioxide, light anhydrous silicic acid, sodium stearyl fumarate, and talc; and even more preferably at least one selected from the group consisting of hydrous silicon dioxide, light anhydrous silicic acid, and talc.

It is particularly preferable that the orally disintegrating tablet of the present disclosure includes hydrous silicon dioxide in the underlying layer, from the viewpoint of storage stability of the content of the active pharmaceutical ingredient.

The thickness of the intermediate layer is preferably 15 µm or less, more preferably 12.5 µm or less, and even more preferably 10 µm or less, from the viewpoint that the bitter taste attributed to butylscopolamine bromide can be concealed more effectively. Furthermore, the thickness of the intermediate layer is preferably 1 µm or more, more preferably 3 µm or more, and even more preferably 5 µm or more, from the viewpoints of productivity and concealment of the bitter taste attributed to butylscopolamine bromide.

In a case in which the intermediate layer is a multilayer of two or more layers, the thickness of the intermediate layer refers to the total thickness of all the layers that form the intermediate layer.

Generally, it is considered that as the thickness of the layer that conceals a bitter taste is thicker, the effect of concealing the bitter taste is provided more effectively. According to the present disclosure, it is surprising that as the thickness of the intermediate layer is made thin, specifically, as the intermediate layer is produced to have a thickness smaller than or equal to a particular thickness, the bitter taste attributed to butylscopolamine bromide can be concealed more significantly.

It is preferable that the intermediate layer covers the entire surface of the active pharmaceutical ingredient-containing core, from the viewpoint of enhancing productivity and concealing the bitter taste attributed to butylscopolamine bromide.

The proportion occupied by the intermediate layer in a fine granule is not particularly limited, and the proportion can be appropriately adjusted according to the purpose.

### <Bitterness-masking layer>

The bitterness-masking layer according to the present disclosure includes talc and at least one water-insoluble polymer.

The bitterness-masking layer is positioned on the outer side of the intermediate layer, with the active pharmaceutical ingredient-containing core being positioned at the center.

Since the bitterness-masking layer includes at least one water-insoluble polymer and talc, dissolubility of the intermediate layer is controlled, and the bitter taste attributed to butylscopolamine bromide can be concealed.

It is desirable that the bitterness-masking layer is in a state of covering at least a portion of the surface of the intermediate layer that covers the active pharmaceutical ingredient-containing core. It is preferable that the bitterness-masking layer covers 1/4 or more, and more preferably 1/2 or more, of the surface of the intermediate layer, and from the viewpoint that the effects of the invention can be provided more noticeably, it is most preferable that the bitterness-masking layer covers the entire surface of the intermediate layer.

### (Talc)

The bitterness-masking layer includes talc.

The content of talc in the bitterness-masking layer is preferably from 5% by mass to 70% by mass, more preferably from 10% by mass to 50% by mass, and even more preferably from 20% by mass to 40% by mass, with respect to the total solid content of the water-insoluble polymer, from the viewpoint of productivity.

### (Water-insoluble polymer)

The bitterness-masking layer includes at least one water-insoluble polymer.

The water-insoluble polymer is not particularly limited as long as it is a pharmacologically acceptable component, and the bitterness-masking layer may include all of known water-insoluble polymers.

According to the present specification, the "water-insoluble polymer" included in the bitterness-masking layer means a polymer having a solubility in water at 20°C of less than 10 g/L.

Examples of the water-insoluble polymer include a water-insoluble cellulose ether and a water-insoluble acrylic acid copolymer.

Examples of the water-insoluble cellulose ether include ethyl cellulose.

Examples of the water-insoluble acrylic acid copolymer include an aminoalkyl methacrylate copolymer RS, an ethyl acrylate/methyl methacrylate copolymer, and a methyl acrylate/methyl methacrylate copolymer.

From the viewpoint of concealing the bitter taste attributed to butylscopolamine bromide, the water-insoluble polymer is preferably a water-insoluble acrylic acid copolymer, and it is more preferable that the water-insoluble polymer includes at least one selected from the group consisting of an aminoalkyl methacrylate copolymer RS and an ethyl acrylate/methyl methacrylate copolymer.

The bitterness-masking layer may include one kind of a water-insoluble polymer, or may include two or more kinds thereof. In a case in which the bitterness-masking layer includes two or more kinds of water-insoluble polymers, from the viewpoint of concealing the bitter taste attributed to butylscopolamine bromide, it is preferable that the bitterness-masking layer includes an aminoalkyl methacrylate copolymer RS and an ethyl acrylate/methyl methacrylate copolymer.

As the water-insoluble polymer, a commercially available product may be used.

Examples of commercially available products of a water-insoluble cellulose ether include an aqueous dispersion liquid of ethyl cellulose (trade name: AQUACOAT (registered trademark) ECD, FMC Corporation).

Examples of commercially available products of a water-insoluble acrylic acid copolymer include an aminoalkyl methacrylate copolymer RS (trade name: EUDRAGIT (registered trademark) RS30D, an ethyl acrylate/methyl methacrylate/trimethylammoniumethyl methacrylate chloride copolymer, dispersion liquid, Evonik Industries AG), an aminoalkyl methacrylate copolymer RS (trade name: EUDRAGIT (registered trademark) RL30D, an ethyl acrylate/methyl methacrylate/trimethylammoniumethyl methacrylate chloride copolymer, dispersion liquid, Evonik Industries AG), and an ethyl acrylate/methyl methacrylate copolymer (trade name: EUDRAGIT (registered trademark) NE30D, dispersion liquid, Evonik Industries AG).

The content of the water-insoluble polymer in the bitterness-masking layer is not particularly limited. For example, from the viewpoint of concealing the bitter taste attributed to butylscopolamine bromide, the content of the water-insoluble polymer in the bitterness-masking layer is preferably from 50% by mass to 80% by mass, more preferably from 55% by mass to 75% by mass, and even more preferably from 55% by mass to 70% by mass, with respect to the total mass of the components that form the bitterness-masking layer.

### (Triethyl citrate)

The bitterness-masking layer may include triethyl citrate.

When the bitterness-masking layer includes triethyl citrate, productivity can be further enhanced.

In a case in which the bitterness-masking layer includes triethyl citrate, the content of triethyl citrate in the bitterness-masking layer is not particularly limited. For example, from the viewpoint of productivity, the content of triethyl citrate in the bitterness-masking layer is preferably from 5% by mass to 15% by mass, and more preferably from 5% by mass to 10% by mass, with respect to the total solid content of the water-insoluble polymer.

### (Other components)

The bitterness-masking layer may include other components as necessary, in addition to the water-insoluble polymer, talc, and triethyl citrate as an optional component, to the extent that the effects of the invention are not impaired.

Examples of the other components include a water-soluble polymer, an excipient, a disintegrant, a lubricating agent, and a binder. These other components can be appropriately selected according to the purpose.

Examples of the water-soluble polymer include a water-soluble cellulose derivative, a water-soluble vinyl polymer derivative, a water-soluble acrylic acid copolymer, and a polyhydric alcohol polymer.

The water-soluble polymer may be any polymer that can dissolve in any of acidic, neutral and alkaline aqueous solutions, may be a gastric polymer that dissolves in an acidic aqueous solution but does not dissolve in a basic aqueous solution, or may be an enteric polymer that dissolves in a basic aqueous solution but does not dissolve in an acidic aqueous solution.

The excipient, disintegrant, and binder for the bitterness-masking layer have the same meanings as the other components explained in the section concerning the active pharmaceutical ingredient-containing core, and therefore, further explanation will not be provided here.

A lubricating agent can contribute to an enhancement of productivity.

The lubricating agent is not particularly limited as long as it is a component that can function as a lubricating agent and is a pharmacologically acceptable component, and any known lubricating agent can be used.

Examples of the lubricating agent include light anhydrous silicic acid, sodium stearyl fumarate, stearic acid, magnesium stearate, calcium stearate, and glycerin monostearate.

In a case in which the bitterness-masking layer includes other components, the bitterness-masking layer may include one kind of other components, or may include two or more kinds thereof.

The content of the other components in the bitterness-masking layer is not particularly limited, and the content can be appropriately set according to the contents of the water-insoluble polymer, talc, and triethyl citrate, which is an optional component.

The thickness of the bitterness-masking layer is not particularly limited. For example, the thickness of the bitterness-masking layer is preferably from 5 µm to 50 µm, more preferably from 10 µm to 45 µm, and even more preferably from 20 µm to 40 µm.

The proportion occupied by the bitterness-masking layer in the fine granule is not particularly limited. For example, the proportion occupied by the bitterness-masking layer in the fine granule is preferably from 15% by mass to 50% by mass, more preferably from 20% by mass to 45% by mass, and even more preferably from 25% by mass to 40% by mass, with respect to the mass of the fine granule.

### <Other layers>

The fine granule according to the present disclosure may include an overcoat layer as a layer on the outer side of the bitterness-masking layer (for example, the outermost layer). It is preferable that the orally disintegrating tablet of the present disclosure further has an overcoat layer including an anionic substance having a pKa of 3.5 or lower. The pKa of the anionic substance is more preferably from 1.9 to 3.5.

Examples of the anionic substance having a pKa of 3.5 or lower include sodium lauryl sulfate (pKa: 1.9) and sodium alginate (pKa: 3). Among them, from the viewpoint of further enhancing the effect of concealing the bitter taste attributed to butylscopolamine bromide, it is more preferable that the overcoat layer includes sodium lauryl sulfate as the anionic substance having a pKa of 3.5 or lower.

Furthermore, from the viewpoint of further suppressing adhesion or aggregation between fine granules during the production process, it is preferable that the overcoat layer includes mannitol.

### <Average particle size of fine granule>

The average particle size of the fine granule is not particularly limited. For example, the average particle size of the fine granule is preferably from 100 µm to 500 µm, more preferably from 100 µm to 400 µm, and even more preferably from 100 µm to 300 µm.

When the average particle size of the fine granule is adjusted to be in the range described above, surface roughness in the oral cavity that occurs at the time of taking the orally disintegrating tablet can be reduced, and therefore, deterioration of the feel of taking the orally disintegrating tablet can be avoided. Also, the orally disintegrating tablet can be produced satisfactorily.

### <Content of fine granule>

It is preferable that the orally disintegrating tablet of the present disclosure includes fine granules such that from 5 mg to 15 mg of butylscopolamine bromide is included per tablet of the orally disintegrating tablet.

### <<Excipient components>>

The orally disintegrating tablet of the present disclosure includes excipient components (hereinafter may be simply referred to as "excipient components") on the outside of the fine granules. The "excipient components" as used herein are components that can contribute to formability and ingestibility of the tablet including fine granules. The excipient components may include, as additives for a pharmacologically acceptable preparation, an excipient, a disintegrant, a lubricating agent, a binder, a fluidizer, a sweetener, a fragrance, a coloring matter, a bitterness inhibitor, an odor adsorbent, and the like, to the extent that the effects of the invention are not impaired. The additives for a preparation may be such that one component performs two or more functions.

The excipient is not particularly limited as long as it is a component that can function as an excipient and is a pharmacologically acceptable component, and any known excipient can be used.

Examples of the excipient include a saccharide, a sugar alcohol, a starch, crystalline cellulose, ethyl cellulose, anhydrous calcium phosphate, and magnesium aluminometasilicate.

Examples of the saccharide include lactose, sucrose, maltose, trehalose, and dextrin. Examples of the sugar alcohol include mannitol, erythritol, isomalt, lactitol, maltitol, sorbitol, and xylitol. Examples of the starch include corn starch, potato starch, rice starch, and wheat starch.

Among these, from the viewpoint of solubility of the orally disintegrating tablet, the excipient is preferably at least one component selected from D-mannitol and erythritol, and D-mannitol is more preferred.

The disintegrant is not particularly limited as long as it is a component that can function as a disintegrant and is a pharmacologically acceptable component, and any known disintegrant can be used.

Examples of the disintegrant include starches such as corn starch and potato starch; partially gelatinzed starch, carboxymethyl starch sodium, carmellose, carmellose calcium, croscarmellose sodium, crospovidone, low-substituted hydroxypropyl cellulose, crystalline cellulose, hydroxypropyl starch, and sodium starch glycolate.

Among these, from the viewpoint of disintegrability of the orally disintegrating tablet, the disintegrant is preferably at least one component selected from the group consisting of crospovidone, croscarmellose sodium, and low-substituted hydroxypropyl cellulose, and is more preferably crospovidone.

The binder is not particularly limited as long as it is a component that can function as a binder and is a pharmacologically acceptable component, and any known binder can be used.

Examples of the binder include hydroxypropyl cellulose, hydroxypropyl methylcellulose, a carboxyvinyl polymer, carmellose sodium, polyethylene glycol, polyvinylpyrrolidone, polyvinyl alcohol, gum arabic, gelatin, gelatinized starch, and pullulan.

The lubricating agent is not particularly limited as long as it is a component that can function as a lubricating agent and is a pharmacologically acceptable component, and any known lubricating agent can be used.

Examples of the lubricating agent include light anhydrous silicic acid, sodium stearyl fumarate, magnesium stearate, calcium stearate, glycerin monostearate, and talc.

Among these, from the viewpoint of productivity of the orally disintegrating tablet, the lubricating agent is preferably sodium stearyl fumarate.

The orally disintegrating tablet of the present disclosure may include one kind of excipient component, or may include two or more kinds thereof.

The content of the excipient components in the orally disintegrating tablet of the present disclosure is not particularly limited, and the content can be appropriately set by considering the type of the active ingredient, the content of the active ingredient, the dose per day of the active ingredient, the particle size of the fine granules, and the like.

It is preferable for the orally disintegrating tablet of the present disclosure that the excipient components included on the outside of the fine granules include an anionic substance having a pKa of 3.5 or lower. When the excipient components include an anionic substance having a pKa of 3.5 or lower, the bitter taste attributed to butylscopolamine bromide can be concealed more noticeably. It is preferable that the pKa of the anionic substance is from 1.9 to 3.5.

Examples of the anionic substance having a pKa of 3.5 or lower include sodium lauryl sulfate (pKa: 1.9) and sodium alginate (pKa: 3). Among them, from the viewpoint that the effect of concealing the bitter taste attributed to butylscopolamine bromide can be further enhanced, it is more preferable that the excipient components include sodium lauryl sulfate as the anionic substance having a pKa of 3.5 or lower.

It is preferable that the anionic substance having a pKa of 3.5 or lower is included to the extent that the effect of concealing a bitter taste can be manifested, and for example, the content is preferably from 0.01% by mass to 20% by mass, more preferably from 0.1% by mass to 10% by mass, and even more preferably from 0.1% by mass to 5% by mass, with respect to the mass of the orally disintegrating tablet.

Furthermore, the orally disintegrating tablet of the present disclosure may also include other active ingredients in addition to butylscopolamine bromide on the outside of the fine granules.

### <Shape of orally disintegrating tablet>

The shape of the orally disintegrating tablet of the present disclosure is not particularly limited as long as the shape is pharmaceutically acceptable. The shape of the orally disintegrating tablet of the present disclosure may be, for example, a circular tablet, or may be a heteromorphic tablet. The shape can be set as appropriate in consideration of the medication compliance.

### <Size of orally disintegrating tablet>

The size of the orally disintegrating tablet of the present disclosure is not particularly limited as long as the size is pharmaceutically acceptable. Generally, when it is considered that orally disintegrating tablets are used for patients who have difficulties in swallowing in many cases, it is preferable that the size of the orally disintegrating tablet of the present disclosure is made as small as possible, while efficacy is taken into consideration.

### <Intraoral disintegration time for orally disintegrating tablet>

The intraoral disintegration time for the orally disintegrating tablet of the present disclosure is not particularly limited. For example, the intraoral disintegration time for the orally disintegrating tablet is preferably less than 60 seconds, and more preferably less than 30 seconds, from the viewpoint of medication compliance.

The term "intraoral disintegration time" according to the present specification refers to the tablet disintegration time that is measured when purified water at 37°C is dropped on an orally disintegrating tablet at a rate of 6 mL/min by using an orally disintegrating tablet analyzer (product name: TRICORPTESTER, manufactured by Okada Seiko Co., Ltd.).

### [Method for producing orally disintegrating tablet]

Regarding the method for producing an orally disintegrating tablet of the present disclosure, any method is acceptable as long as the orally disintegrating tablet of the present disclosure that is configured as described above can be produced, and there are no particular limitations. The orally disintegrating tablet of the present disclosure can be suitably produced by, for example, the following method.

The method for producing an orally disintegrating tablet of the present disclosure is a production method including a step for obtaining fine granules (hereinafter, appropriately referred to as "fine granule production step") by a production method that includes Step A of coating core particles with an active pharmaceutical ingredient layer by spraying a spray liquid for an active pharmaceutical ingredient layer, which includes butylscopolamine bromide and water-insoluble particles, onto core particles that each serve as a central core of an active pharmaceutical ingredient-containing core (hereinafter, appropriately referred to as "Step A"); Step B of coating the active pharmaceutical ingredient-containing core with an intermediate layer by spraying a spray liquid for an intermediate layer, which includes water-insoluble particles, onto the active pharmaceutical ingredient-containing core that is obtained by coating the core particles with an active pharmaceutical ingredient layer (hereinafter, appropriately referred to as "Step B"); and Step C of further coating the active pharmaceutical ingredient-containing core with a bitterness-masking layer by spraying a spray liquid for a bitterness-masking layer, which includes talc and at least one water-insoluble polymer, onto the active pharmaceutical ingredient-containing core that is coated with at least the intermediate layer (hereinafter, appropriately referred to as "Step C"), and a step for mixing the fine granules thus obtained with excipient components (hereinafter, appropriately referred to as "mixing step").

In the following description, the various steps of the method for producing an orally disintegrating tablet of the present disclosure will be explained in detail.

Since the types of the components used in the various steps, and preferred embodiments are as described above in the sections concerning the fine granules and the excipient components, further explanation will not be provided here.

### <<Fine granule production step>>

The method for producing an orally disintegrating tablet of the present disclosure includes a step of obtaining fine granules (fine granule production step) by a production method that includes Step A of coating core particles with an active pharmaceutical ingredient layer by spraying a spray liquid for an active pharmaceutical ingredient layer, which includes butylscopolamine bromide and water-insoluble particles, onto core particles that each serve as a central core of an active pharmaceutical ingredient-containing core; Step B of coating the active pharmaceutical ingredient-containing core with an intermediate layer by spraying a spray liquid for an intermediate layer, which includes water-insoluble particles, onto the active pharmaceutical ingredient-containing core that is obtained by coating the core particles with an active pharmaceutical ingredient layer; and Step C of further coating the active pharmaceutical ingredient-containing core with a bitterness-masking layer by spraying a spray liquid for a bitterness-masking layer, which includes talc and at least one water-insoluble polymer, onto the active pharmaceutical ingredient-containing core that is coated with at least an intermediate layer.

When a conventional method, for example, a method of coating butylscopolamine bromide with the layers disclosed in WO2005/105045A, is employed in order to conceal the bitter taste of butylscopolamine bromide, aggregation of particles including butylscopolamine bromide occurs, and it is difficult to produce fine granules.

When aggregation of particles occurs, coarse particles having a size larger than or equal to the intended particle size are generated, and therefore, there is a problem that yield is decreased. In order to suppress aggregation of particles, it may be considered to decrease the spray velocity of the spray liquid; however, production time is increased. As such, aggregation of particles brings about a decrease in production efficiency.

As the method for producing an orally disintegrating tablet of the present disclosure includes the above-described step for obtaining fine granules (fine granule production step) by a production method including Step A, Step B, and Step C, an orally disintegrating tablet that conceals the bitter taste attributed to butylscopolamine bromide can be produced highly efficiently.

### <Step A>

Step A is a step of coating core particles with an active pharmaceutical ingredient layer by spraying a spray liquid for an active pharmaceutical ingredient layer, which includes butylscopolamine bromide and water-insoluble particles, onto core particles that each serve as a central core of an active pharmaceutical ingredient-containing core.

In Step A, an active pharmaceutical ingredient-containing core in which a core particle is coated with an active pharmaceutical ingredient layer including butylscopolamine bromide and water-insoluble particles, is granulated.

Since the spray liquid for an active pharmaceutical ingredient layer includes water-insoluble particles together with butylscopolamine bromide, when the active pharmaceutical ingredient-containing core is granulated by coating core particles with an active pharmaceutical ingredient layer, aggregation of the active pharmaceutical ingredient-containing cores that include butylscopolamine bromide is suppressed. Therefore, the production efficiency for the active pharmaceutical ingredient-containing core is increased.

The spray liquid for an active pharmaceutical ingredient layer may include, in addition to butylscopolamine bromide and water-insoluble particles, other components such as an excipient, a disintegrant and a binder as necessary, to the extent that the effects of the invention are not impaired.

In regard to the water-insoluble particles included in the spray liquid for an active pharmaceutical ingredient layer, from the viewpoint of noticeably suppressing aggregation of the active pharmaceutical ingredient-containing cores that include butylscopolamine bromide, and further increasing the production efficiency for the active pharmaceutical ingredient-containing core, it is preferable that the water-insoluble particles are formed from at least one selected from the group consisting of hydrous silicon dioxide, light anhydrous silicic acid, sodium stearyl fumarate, magnesium stearate, and talc; more preferably at least one selected from the group consisting of hydrous silicon dioxide, light anhydrous silicic acid, sodium stearyl fumarate, and talc; and even more preferably at least one selected from the group consisting of hydrous silicon dioxide, light anhydrous silicic acid, and talc.

In regard to the method for producing an orally disintegrating tablet of the present disclosure, from the viewpoint of storage stability of the content of the active pharmaceutical ingredient, it is particularly preferable that the spray liquid for an active pharmaceutical ingredient layer includes hydrous silicon dioxide.

For the preparation of the spray liquid for an active pharmaceutical ingredient layer, it is preferable to use water, and a mixed solvent of water and a solvent that is miscible with water may also be used to the extent that the effects of the invention are not impaired. Examples of the solvent that is miscible with water include pharmacologically acceptable solvents, including alcohols such as methanol, ethanol, propanol and isopropanol; acetone, and acetonitrile.

The conditions such as temperature employed at the time of preparing the spray liquid for an active pharmaceutical ingredient layer are not particularly limited. The spray liquid for an active pharmaceutical ingredient layer can be prepared by, for example, adding butylscopolamine bromide, water-insoluble particles, and other components as necessary, to water that has been set to a temperature of from 20°C to 80°C.

The viscosity, temperature and the like of the spray liquid for an active pharmaceutical ingredient layer can be appropriately set in accordance with the types and amounts of the various components that are incorporated into the spray liquid for an active pharmaceutical ingredient layer.

The amount of the spray liquid for an active pharmaceutical ingredient layer that is sprayed onto the core particles is not particularly limited as long as it is an amount that provides a form in which the core particles are coated with an active pharmaceutical ingredient layer.

For example, it is desirable to spray the spray liquid for an active pharmaceutical ingredient layer in an amount that includes butylscopolamine bromide in an amount, on a mass basis, of from 0.01 times to 100 times, from 0.05 times to 50 times, or from 0.1 times to 20 times, the total mass of the core particles, onto the core particles.

The form in which the core particles are coated with the active pharmaceutical ingredient layer may be in a state in which the spray liquid for an active pharmaceutical ingredient layer covers at least a portion of the surface of the core particles. It is preferable that the spray liquid for an active pharmaceutical ingredient layer covers 1/4 or more, and more preferably 1/2 or more, of the surface of the core particles, and it is most preferable that the spray liquid covers the entire surface of the core particles.

The spray liquid for an active pharmaceutical ingredient layer may infiltrate into the core particles by coating, and the core particle may be in a form in which butylscopolamine bromide exists up to the interior of the core particle through this infiltration.

Spraying of the spray liquid for an active pharmaceutical ingredient layer onto the core particles can be carried out using a granulator. The method of spraying the spray liquid for an active pharmaceutical ingredient layer onto the core particles using a granulator is not particularly limited, and the method can be appropriately set in accordance with the amount of the core particles that serve as an object of spraying of the spray liquid for an active pharmaceutical ingredient layer, physical strength of the core particles, and the like.

Examples of the granulator that is used when the spray liquid for an active pharmaceutical ingredient layer is sprayed onto the core particles include granulators such as a fluidized bed granulator, a tumbling fluidized bed granulator, a spouted fluidized bed granulator, and a compound mechanical agitation type fluidized bed granulator.

Examples of the fluidized bed granulator include fluidized bed granulators (product name: MP-01 (FD), MP-01 (SPC), and the like, manufactured by Powrex Corporation), and flow coaters (product name: FL-1, manufactured by Freund Corporation).

The spray velocity, spraying time and the like of the spray liquid for an active pharmaceutical ingredient layer are not particularly limited, and can be appropriately set in accordance with the content of butylscopolamine bromide in the spray liquid for an active pharmaceutical ingredient layer, the viscosity of the spray liquid for an active pharmaceutical ingredient layer, and the like.

The charge air temperature of the gas supplied into the granulator is, for example, preferably from 25°C to 100°C, more preferably from 30°C to 80°C, and even more preferably from 30° to 60°C.

The air supply rate of the gas supplied into the granulator is, for example, preferably from 0.3 m³/h to 1.0 m³/h, more preferably from 0.5 m³/h to 0.9 m³/h, and even more preferably from 0.5 m³/h to 0.8 m³/h.

### <Step B>

Step B is a step of coating the active pharmaceutical ingredient-containing core with an intermediate layer by spraying a spray liquid for an intermediate layer, which includes water-insoluble particles, onto the active pharmaceutical ingredient-containing core that is obtained by coating the core particles with an active pharmaceutical ingredient layer.

In Step B, a granulated material in which the active pharmaceutical ingredient-containing core is coated with an intermediate layer that includes water-insoluble particles can be obtained.

In regard to Step B, when the active pharmaceutical ingredient-containing core that includes butylscopolamine bromide is coated with an intermediate layer, the bitter taste attributed to butylscopolamine bromide can be concealed. Furthermore, since the spray liquid for an intermediate layer includes water-insoluble particles, when the active pharmaceutical ingredient-containing core that includes butylscopolamine bromide is coated with an intermediate layer, the occurrence of aggregation is suppressed, and therefore, production efficiency is increased.

The spray liquid for an intermediate layer may include, in addition to the water-insoluble particles, other components such as an excipient, a disintegrant, and a binder as necessary, to the extent that the effects of the invention are not impaired.

From the viewpoint of noticeably suppressing the occurrence of aggregation when the active pharmaceutical ingredient-containing core that includes butylscopolamine bromide is coated with an intermediate layer, and from the viewpoint of further increasing the production efficiency, it is preferable that the water-insoluble particles included in the spray liquid for an intermediate layer are formed from at least one selected from the group consisting of hydrous silicon dioxide, light anhydrous silicic acid, sodium stearyl fumarate, magnesium stearate, and talc; more preferably at least one selected from the group consisting of hydrous silicon dioxide, light anhydrous silicic acid, sodium stearyl fumarate, and talc; and even more preferably at least one selected from the group consisting of hydrous silicon dioxide, light anhydrous silicic acid, and talc.

In regard to the method for producing an orally disintegrating tablet of the present disclosure, from the viewpoint of storage stability of the content of the active pharmaceutical ingredient, it is particularly preferable that the spray liquid for an intermediate layer includes hydrous silicon dioxide.

The concentration of the water-insoluble particles in the spray liquid for an intermediate layer is not particularly limited. For example, the concentration of the water-insoluble particles in the spray liquid for an intermediate layer is preferably from 0.5% by mass to 10.0% by mass, more preferably from 1.0% by mass to 8.0% by mass, and even more preferably from 1.5% by mass to 5.0% by mass.

When the concentration of the water-insoluble particles in the spray liquid for an intermediate layer is adjusted to the in the range described above, the occurrence of aggregation can be sufficiently suppressed.

Meanwhile, in a case in which the intermediate layer is composed of a plurality of layers of two or more layers, it is preferable that the content of the water-insoluble particles is in the range described above with respect to the total mass of the spray liquids for forming the various layers.

For the preparation of the spray liquid for an intermediate layer, it is preferable to use water, and a mixed solvent of water and a solvent that is miscible with water may also be used to the extent that the effects of the invention are not impaired. Examples of the solvent that is miscible with water include pharmacologically acceptable solvents, including alcohols such as methanol, ethanol, propanol, and isopropanol; acetone, and acetonitrile.

The conditions such as temperature employed at the time of preparing the spray liquid for an intermediate layer are not particularly limited. The spray liquid for an intermediate layer can be prepared by, for example, adding the water-insoluble particles and other components as necessary to water that has been set to a temperature of from 20°C to 80°C.

The viscosity, temperature and the like of the spray liquid for an intermediate layer can be appropriately set in accordance with the types and amounts of the various components incorporated into the spray liquid for an intermediate layer.

The amount of the spray liquid for an intermediate layer that is sprayed onto the active pharmaceutical ingredient-containing core is not particularly limited as long as it is an amount that provides a form in which the active pharmaceutical ingredient-containing core is coated with an intermediate layer.

The form in which the active pharmaceutical ingredient-containing core is coated with an intermediate layer may be in a state in which the spray liquid for an intermediate layer covers at least a portion of the surface of the active pharmaceutical ingredient-containing core. It is preferable that the spray liquid for an intermediate layer covers 1/4 or more, and more preferably 1/2 or more, of the surface of the active pharmaceutical ingredient-containing core, and from the viewpoint that the effects of the invention can be provided more noticeably, it is most preferable that the intermediate layer covers the entire surface of the active pharmaceutical ingredient-containing core.

Spraying of the spray liquid for an intermediate layer onto the active pharmaceutical ingredient-containing core can be carried out using a granulator. The method of spraying the spray liquid for an intermediate layer onto the active pharmaceutical ingredient-containing core using a granulator is not particularly limited, and can be appropriately set in accordance with the amount of the active pharmaceutical ingredient-containing core that serves as an object of spraying of the spray liquid for an intermediate layer, physical strength of the active pharmaceutical ingredient-containing core, and the like.

Since the examples of the granulator and the fluidized bed granulator used when the spray liquid for an intermediate layer is sprayed onto the active pharmaceutical ingredient-containing core are similar to those for Step A, further explanation will not be provided here.

The spray velocity, spraying time and the like of the spray liquid for an intermediate layer are not particularly limited, and can be appropriately set in accordance with the content of the water-insoluble particles in the spray liquid for an intermediate layer, the viscosity of the spray liquid for an intermediate layer, and the like.

Since the charge air temperature and the air supply rate of the gas supplied into the granulator are similar to those for Step A, further explanation will not be provided here.

In the method for producing an orally disintegrating tablet of the present disclosure, from the viewpoint of increasing the production efficiency, it is preferable that Step B includes Step B1 of coating the active pharmaceutical ingredient-containing core with an underlying layer by spraying a spray liquid for an underlying layer, which includes water-insoluble particles, onto the active pharmaceutical ingredient-containing core (hereinafter, appropriately referred to as "Step B1"); and Step B2 of further coating the active pharmaceutical ingredient-containing core with an overlying layer by spraying a spray liquid for an overlying layer, which includes water-insoluble particles, onto the active pharmaceutical ingredient-containing core that has been coated with at least an underlying layer (hereinafter, appropriately referred to as "Step B2").

### <Step B1>

Step B1 is a step of coating the active pharmaceutical ingredient-containing core with an underlying layer by spraying a spray liquid for an underlying layer, which includes water-insoluble particles, onto the active pharmaceutical ingredient-containing core.

In Step B1, a granulated material in which the active pharmaceutical ingredient-containing core is coated with an underlying layer can be obtained.

Since the spray liquid for an underlying layer includes water-insoluble particles, when the active pharmaceutical ingredient-containing core that includes butylscopolamine bromide is coated with an underlying layer, the occurrence of aggregation is suppressed, and therefore, production efficiency is increased.

The spray liquid for an underlying layer may include, in addition to the water-insoluble particles, other components such as an excipient, a disintegrant, and a binder as necessary, to the extent that the effects of the invention are not impaired.

From the viewpoint of noticeably suppressing the occurrence of aggregation when the active pharmaceutical ingredient-containing core that includes butylscopolamine bromide is coated with the underlying layer, and from the viewpoint of further increasing the production efficiency, it is preferable that the water-insoluble particles included in the spray liquid for an underlying layer is formed from at least one selected from the group consisting of hydrous silicon dioxide, light anhydrous silicic acid, sodium stearyl fumarate, and talc; and more preferably at least one selected from the group consisting of hydrous silicon dioxide, light anhydrous silicic acid, and talc.

In regard to the method for producing an orally disintegrating tablet of the present disclosure, from the viewpoint of storage stability of the content of the active pharmaceutical ingredient, it is more preferable that the spray liquid for an underlying layer includes hydrous silicon dioxide.

The content of the water-insoluble particles in the spray liquid for an underlying layer is not particularly limited. For example, the content of the water-insoluble particles in the spray liquid for an underlying layer is preferably from 0.5% by mass to 80.0% by mass, more preferably from 1.0% by mass to 60.0% by mass, and even more preferably from 1.0% by mass to 50.0% by mass, with respect to the total amount of the spray liquid.

When the content of the water-insoluble particles in the spray liquid for an underlying layer is adjusted to be in the range described above, the occurrence of aggregation is noticeably suppressed, and therefore, production efficiency can be further increased.

For the preparation of the spray liquid for an underlying layer, it is preferable to use water, and a mixed solvent of water and a solvent that is miscible with water may also be used to the extent that the effects of the invention are not impaired. Examples of the solvent that is miscible with water include pharmacologically acceptable solvents, including alcohols such as methanol, ethanol, propanol and isopropanol; acetone, and acetonitrile.

The conditions such as temperature employed at the time of preparing the spray liquid for an underlying layer are not particularly limited. The spray liquid for an underlying layer can be prepared by, for example, adding the water-insoluble particles and other components as necessary to water that has been set to a temperature of from 20°C to 80°C.

The viscosity, temperature and the like of the spray liquid for an underlying layer can be appropriately set in accordance with the types and amounts of the various components that are incorporated into the spray liquid for an underlying layer.

The amount of the spray liquid for an underlying layer that is sprayed onto the active pharmaceutical ingredient-containing core is not particularly limited as long as it is an amount that provides a form in which the active pharmaceutical ingredient-containing core is coated with the underlying layer.

The form in which the active pharmaceutical ingredient-containing core is coated with the underlying layer may be in a state in which the spray liquid for an underlying layer covers at least a portion of the surface of the active pharmaceutical ingredient-containing core. It is preferable that the spray liquid for an underlying layer covers 1/4 or more, and more preferably 1/2 or more, of the surface of the active pharmaceutical ingredient-containing core, and from the viewpoint that the effects of the invention can be provided more noticeably, it is most preferable that the spray liquid for an underlying layer covers the entire surface of the active pharmaceutical ingredient-containing core.

Spraying of the spray liquid for an underlying layer onto the active pharmaceutical ingredient-containing core can be carried out using a granulator. The method of spraying the spray liquid for an underlying layer onto the active pharmaceutical ingredient-containing core using a granulator is not particularly limited, and can be appropriately set in accordance with the amount of the active pharmaceutical ingredient-containing core that serves as an object of spraying of the spray liquid for an underlying layer, physical strength of the active pharmaceutical ingredient-containing core, and the like.

Since the examples of the granulator and the fluidized bed granulator used at the time of spraying the spray liquid for an underlying layer onto the active pharmaceutical ingredient-containing core are similar to those for Step A, further explanation will not be provided here.

The spray velocity, spraying time and the like of the spray liquid for an underlying layer are not particularly limited, and can be appropriately set in accordance with the content of the water-insoluble particles in the spray liquid for an underlying layer, the viscosity of the spray liquid for an underlying layer, and the like.

Since the charge air temperature and the air supply rate of the gas supplied into the granulator are similar to those for Step A, further explanation will not be provided here.

### <Step B2>

Step B2 is a step of further coating the active pharmaceutical ingredient-containing core with an overlying layer by spraying a spray liquid for an overlying layer, which includes water-insoluble particles, onto the active pharmaceutical ingredient-containing core that has been coated with at least an underlying layer.

In Step B2, a granulated material in which the active pharmaceutical ingredient-containing core coated with at least an underlying layer (hereinafter, appropriately referred to as "underlying layer-coated active pharmaceutical ingredient-containing core") is further coated with an overlying layer that includes water-insoluble particles, can be obtained.

Examples of the material for the water-insoluble particles that are included in the spray liquid for an overlying layer include lubricating agents such as hydrous silicon dioxide, light anhydrous silicic acid, sodium stearyl fumarate, magnesium stearate, calcium stearate, glycerin monostearate, and talc.

The spray liquid for an overlying layer may include, in addition to the water-insoluble particles, other components such as an excipient, a disintegrant, and a binder as necessary, to the extent that the effects of the invention are not impaired.

The content of the water-insoluble particles in the spray liquid for an overlying layer is not particularly limited. For example, from the viewpoint of increasing the production efficiency, the content of the water-insoluble particles in the spray liquid for an overlying layer is preferably from 0.1% by mass to 10.0% by mass, more preferably from 0.5% by mass to 5.0% by mass, and even more preferably from 1.0% by mass to 3.0% by mass, with respect to the total amount of the spray liquid.

For the preparation of the spray liquid for an overlying layer, it is preferable to use water, and a mixed solvent of water and a solvent that is miscible with water may also be used to the extent that the effects of the invention are not impaired. Examples of the solvent that is miscible with water include pharmacologically acceptable solvents, including alcohols such as methanol, ethanol, propanol and isopropanol; acetone, and acetonitrile.

The conditions such as temperature employed at the time of preparing the spray liquid for an overlying layer are not particularly limited. The spray liquid for an overlying layer can be prepared by, for example, adding the water-insoluble particles and other components as necessary to water that has been set to a temperature of from 20°C to 80°C.

The viscosity, temperature and the like of the spray liquid for an overlying layer can be appropriately set in accordance with the types and amounts of the various components that are incorporated into the spray liquid for an overlying layer.

The amount of the spray liquid for an overlying layer that is sprayed onto the underlying layer-coated active pharmaceutical ingredient-containing core is not particularly limited as long as it is an amount that provides a form in which the underlying layer-coated active pharmaceutical ingredient-containing core is coated with the overlying layer.

The form in which the underlying layer-coated active pharmaceutical ingredient-containing core is coated with the overlying layer may be in a state in which the spray liquid for an overlying layer covers at least a portion of the surface of the underlying layer-coated active pharmaceutical ingredient-containing core. It is preferable that the spray liquid for an overlying layer covers 1/4 or more, and more preferably 1/2 or more, of the surface of the underlying layer-coated active pharmaceutical ingredient-containing core, and it is most preferable that the spray liquid for an overlying layer covers the entire surface of the underlying layer-coated active pharmaceutical ingredient-containing core.

Spraying of the spray liquid for an overlying layer onto the underlying layer-coated active pharmaceutical ingredient-containing core can be carried out using a granulator. The method of spraying the spray liquid for an overlying layer onto the underlying layer-coated active pharmaceutical ingredient-containing core using a granulator is not particularly limited, and can be appropriately set in accordance with the amount of the underlying layer-coated active pharmaceutical ingredient-containing core that serves as an object of spraying of the spray liquid for an overlying layer, physical strength of the underlying layer-coated active pharmaceutical ingredient-containing core, and the like.

Since the examples of the granulator and the fluidized bed granulator used at the time of spraying the spray liquid for an overlying layer onto the underlying layer-coated active pharmaceutical ingredient-containing core are similar to those for Step A, further explanation will not be provided here.

The spray velocity, spraying time and the like of the spray liquid for an overlying layer are not particularly limited, and can be appropriately set in accordance with the content of the water-insoluble particles in the spray liquid for an overlying layer, the viscosity of the spray liquid for an overlying layer, and the like.

Since the charge air temperature and the air supply rate of the gas supplied into the granulator are similar to those for Step A, further explanation will not be provided here.

### <Step C>

Step C is a step of further coating the active pharmaceutical ingredient-containing core with a bitterness-masking layer by spraying a spray liquid for a bitterness-masking layer, which includes talc and at least one water-insoluble polymer, onto the active pharmaceutical ingredient-containing core that has been coated with at least an intermediate layer.

In Step C, a granulated material in which the active pharmaceutical ingredient-containing core that has been coated with at least an intermediate layer (hereinafter, appropriately referred to as "intermediate layer-coated active pharmaceutical ingredient-containing core") is further coated with a bitterness-masking layer that includes talc and at least one water-insoluble polymer.

Since the spray liquid for a bitterness-masking layer includes talc and at least one water-insoluble polymer, when the intermediate layer-coated active pharmaceutical ingredient-containing core is coated with a bitterness-masking layer, the occurrence of aggregation is suppressed, and therefore, production efficiency can be increased.

The water-insoluble polymer that is included in the spray liquid for a bitterness-masking layer is preferably at least one selected from the group consisting of an aminoalkyl methacrylate copolymer RS and an ethyl acrylate/methyl methacrylate copolymer, from the viewpoint of concealing the bitter taste attributed to butylscopolamine bromide.

The concentration of the water-insoluble polymer in the spray liquid for a bitterness-masking layer is not particularly limited. For example, the concentration of the water-insoluble polymer in the spray liquid for a bitterness-masking layer is preferably from 1% by mass to 25% by mass, more preferably from 5% by mass to 20% by mass, and even more preferably from 10% by mass to 15% by mass.

When the concentration of the water-insoluble polymer in the spray liquid for a bitterness-masking layer is adjusted to be in the range described above, the viscosity of the spray liquid does not increase too high, and therefore, enlargement of the liquid droplets of the spray liquid can be suppressed.

The concentration of talc in the spray liquid for a bitterness-masking layer is not particularly limited. For example, the concentration of talc in the spray liquid for a bitterness-masking layer is preferably from 1% by mass to 20% by mass, more preferably from 3% by mass to 15% by mass, and even more preferably 5% by mass to 10% by mass.

When the concentration of talc in the spray liquid for a bitterness-masking layer is adjusted to be in the range described above, the occurrence of aggregation can be suppressed sufficiently.

The spray liquid for a bitterness-masking layer may include other components such as triethyl citrate, a water-soluble polymer, an excipient, a disintegrant, a lubricating agent, and a binder as necessary, in addition to the water-insoluble polymer and talc.

It is preferable that the spray liquid for a bitterness-masking layer includes triethyl citrate.

In a case in which the spray liquid for a bitterness-masking layer includes triethyl citrate, the content of triethyl citrate in the spray liquid for a bitterness-masking layer is preferably from 5% by mass to 15% by mass, more preferably from 8% by mass to 12% by mass, and even more preferably from 9% by mass to 11% by mass, with respect to the total solid content of the water-insoluble polymer.

When the content of triethyl citrate in the spray liquid for a bitterness-masking layer is adjusted to be in the range described above with respect to the total solid content of the water-insoluble polymer, for example, the yield of the fine granules can be further enhanced, and production efficiency can be further increased.

For the preparation of the spray liquid for a bitterness-masking layer, it is preferable to use water, and a mixed solvent of water and a solvent that is miscible with water may also be used to the extent that the effects of the invention are not impaired. Examples of the solvent that is miscible with water include pharmacologically acceptable solvents, including alcohols such as methanol, ethanol, propanol and isopropanol; acetone, and acetonitrile.

The conditions such as temperature employed at the time of preparing the spray liquid for a bitterness-masking layer are not particularly limited. The spray liquid for a bitterness-masking layer can be prepared by, for example, adding talc, at least one water-insoluble polymer, and other components as necessary, to water that has been set to a temperature of from 20°C to 80°C.

The viscosity, temperature and the like of the spray liquid for a bitterness-masking layer can be appropriately set in accordance with the types and amounts of the various components that are incorporated into the spray liquid for a bitterness-masking layer.

The amount of the spray liquid for a bitterness-masking layer that is sprayed onto the intermediate layer-coated active pharmaceutical ingredient-containing core is not particularly limited as long as it is an amount that provides a form in which the intermediate layer-coated active pharmaceutical ingredient-containing core is coated with the bitterness-masking layer.

The form in which the intermediate layer-coated active pharmaceutical ingredient-containing core is coated with a bitterness-masking layer may be in a state in which the spray liquid for a bitterness-masking layer covers at least a portion of the surface of the intermediate layer-coated active pharmaceutical ingredient-containing core. It is preferable that the spray liquid for a bitterness-masking layer covers 1/4 or more, and more preferably 1/2 or more, of the surface of the intermediate layer-coated active pharmaceutical ingredient-containing core, and from the viewpoint that the effects of the invention can be provided more noticeably, it is most preferable that the spray liquid for a bitterness-masking layer covers the entire surface of the intermediate layer-coated active pharmaceutical ingredient-containing core.

Spraying of the spray liquid for a bitterness-masking layer onto the intermediate layer-coated active pharmaceutical ingredient-containing core can be carried out using a granulator. The method of spraying the spray liquid for a bitterness-masking layer onto the intermediate layer-coated active pharmaceutical ingredient-containing core using a granulator is not particularly limited, and can be appropriately set in accordance with the amount of the intermediate layer-coated active pharmaceutical ingredient-containing core that serves as an object of spraying of the spray liquid for a bitterness-masking layer, physical strength of the intermediate layer-coated active pharmaceutical ingredient-containing core, and the like.

Examples of the granulator that is used when the spray liquid for a bitterness-masking layer is sprayed onto the intermediate layer-coated active pharmaceutical ingredient-containing core, include granulators such as a fluidized bed granulator, a tumbling fluidized bed granulator, a spouted fluidized bed granulator, and a compound mechanical agitation type fluidized bed granulator.

Examples of the fluidized bed granulators are similar to those for Step A, and therefore, further explanation will not be provided here.

The spray velocity, spraying time and the like of the spray liquid for a bitterness-masking layer are not particularly limited, and can be appropriately set in accordance with the contents of the water-insoluble polymer and talc in the spray liquid for a bitterness-masking layer, the viscosity of the spray liquid for a bitterness-masking layer, and the like.

Since the charge air temperature and the air supply rate of the gas supplied into the granulator are similar to those for Step A, further explanation will not be provided here.

### <Other steps>

The fine granule production step may include other steps such as a step of forming another layer such as an overcoat layer, in addition to Step A, Step B that may include Step B1 and Step B2, and Step C. An overcoat layer is a layer intended for further suppressing adhesion or aggregation between fine granules, and can be formed by, for example, spraying a spray liquid including mannitol onto an object of coating.

Furthermore, the fine granule production step may also include a drying step as another step.

### (Drying step)

A drying step is a step of removing a solvent such as water that is not included in a spray liquid sprayed onto an object of spraying such as core particles.

The fine granule production step may include a drying step in at least one step among Step A, Step B that may include Step B1 and Step B2, and Step C, or may include between the various steps.

In a case in which the drying step is included in at least one step among Step A, Step B that may include Step B1 and Step B2, and Step C, removal of the solvent can be carried out all together with spraying of a spray liquid onto an object of spraying by using, for example, a fluidized bed granulator.

Regarding the method for removing the solvent that is included in a spray liquid sprayed onto an object of spraying, a method of independently performing drying only may be mentioned, in addition to a method of using a fluidized bed granulator. Examples of the method of independently performing drying only include a method of using a vacuum dryer, and a method of using a hot air dryer.

### <<Mixing step>>

The method for producing an orally disintegrating tablet of the present disclosure includes a step of mixing the fine granules obtained in the fine granule production step described above, with excipient components (mixing step).

The method of mixing the fine granules with excipient components is not particularly limited. Regarding the method of mixing the fine granules with excipient components, a method of performing mixing using a known mixing machine such as a V-type mixing machine (Tsutsui Scientific Instruments Co., Ltd.) or a fluidized bed granulator (Powrex Corporation) may be mentioned.

The conditions such as the time required for mixing can be appropriately adjusted depending on the types of the fine granules and the excipient components.

### <<Other steps>>

The method for producing an orally disintegrating tablet of the present disclosure may also include, in addition to the fine granule production step and the mixing step, other steps such as a step of tableting a mixed powder obtained by mixing the fine granules and the excipient components, and a step of drying the tableted material obtained by tableting a mixed powder.

The method of tableting a mixed powder of the fine granules and the excipient components is not particularly limited. Regarding the method of tableting a mixed powder of the fine granules and the excipient components, a method of tableting by using a tableting machine such as a rotary tableting machine (product name: HT-AP-SS, Hata Tekkosho Co., Ltd.) or a high-speed rotating type tableting machine (product name: AQUARIUS G, Kikusui Seisakusho, Ltd.) may be mentioned. The temperature employed at the time of tableting is not particularly limited, and can be appropriately set.

Regarding the method of drying a tableted material obtained by tableting a mixed powder, a method of performing drying by vacuum drying, fluidized bed drying or the like may be mentioned.

### EXAMPLES

Hereinafter, the present invention will be more specifically described by way of.

### [Production of fine granules]

### <Production Example 1>

400 g of crystalline cellulose (granules) (trade name: CELPHERE (registered trademark) CP-203, particle size range: from 150 µm to 300 µm, Asahi Kasei Chemicals Corporation) as core particles was introduced into a fluidized bed granulator (type: MP-01 (SPC), Powrex Corporation), and the charge air temperature of the fluidized bed granulator was adjusted to 60°C, the air supply rate to from 0.6 m³/h to 1 m³/h, and the liquid velocity to about 2 g/min.

The entire amount of an active pharmaceutical ingredient layer 1A spray liquid, the entire amount of an underlying layer 1A spray liquid, and the entire amount of an overlying layer 1A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 1A / intermediate layer 1A (underlying layer 1A / overlying layer 1A)] were obtained.

### [Active pharmaceutical ingredient layer 1A spray liquid]

| | |
|---|---|
| • Butylscopolamine bromide (active ingredient, antispasmodic agent) | 107.98 g |
| • Hydroxypropyl cellulose (type SSL) 10 mass% aqueous solution | 162.0 g |
| • Light anhydrous silicic acid (water-insoluble particles) | 27.0 g |
| • Purified water | 422.9 g |

### [Underlying layer 1A spray liquid]

| | |
|---|---|
| • Hydroxypropyl cellulose (type SSL) 10 mass% aqueous solution (coating film component) | 24.3 g |
| • Mannitol | 16.2 g |
| • Light anhydrous silicic acid (water-insoluble particles) | 4.0 g |
| • Purified water | 64.3 g |

### [Overlying layer 1A spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 146.3 g |
| • Mannitol | 14.6 g |
| • Talc (water-insoluble particles) | 5.9 g |
| • Purified water | 125.9 g |

400 g of the primary fine granules thus obtained were introduced into a fluidized bed granulator (type: MP-01 (SPC), Powrex Corporation), and the charge air temperature of the fluidized bed granulator was adjusted to 40°C, the air supply rate to from 0.6 m³/h to 1 m³/h, and the liquid velocity to about 4 g/min.

1422 g (defined amount) of a bitterness-masking layer 1A spray liquid and 244.4 g (defined amount) of an overcoat layer 1A spray liquid, all of which had compositions as described below and had been prepared in advance, were spray-coated successively in this order on the primary fine granules, and then drying was performed for 30 minutes by setting the charge air temperature of the fluidized bed granulator to 80°C. Thus, fine granules of Production Example 1 [layer configuration: core particle / active pharmaceutical ingredient layer 1A / intermediate layer 1A (underlying layer 1A / overlying layer 1A) / bitterness-masking layer 1A / overcoat layer 1A] were obtained.

### [Bitterness-masking layer 1A spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 444.4 g |
| • Aminoalkyl methacrylate copolymer RS dispersion liquid (water-insoluble polymer) (trade name: EUDRAGIT (registered trademark) RL30D, 30 mass% aqueous dispersion liquid, Evonik Industries AG) | 444.4 g |
| • Triethyl citrate | 26.7 g |
| • Talc | 133.3 g |
| • Purified water | 1084.4 g |

### [Overcoat layer 1A spray liquid]

| | |
|---|---|
| • Mannitol | 140.0 g |
| • Purified water | 860.0 g |

### <Production Example 2>

400 g of crystalline cellulose (granules) (trade name: CELPHERE (registered trademark) CP-203) as core particles was introduced into a fluidized bed granulator (type: MP-01 (SPC)), and the charge air temperature of the fluidized bed granulator was adjusted to 60°C, the air supply rate to from 0.6 m³/h to 1 m³/h, and the liquid velocity to about 2 g/min.

The entire amount of an active pharmaceutical ingredient layer 2A spray liquid, the entire amount of an underlying layer 2A spray liquid, and the entire amount of an overlying layer 2A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 2A / intermediate layer 2A (underlying layer 2A / overlying layer 2A)] were obtained.

### [Active pharmaceutical ingredient layer 2A spray liquid]

| | |
|---|---|
| • Butylscopolamine bromide (active ingredient, antispasmodic agent) | 107.98 g |
| • Hydroxypropyl cellulose (type SSL) 10 mass% aqueous solution | 162.0 g |
| • Light anhydrous silicic acid (water-insoluble particles) | 27.0 g |
| • Purified water | 422.9 g |

### [Underlying layer 2A spray liquid]

| | |
|---|---|
| • Hydroxypropyl cellulose (type SSL) 10 mass% aqueous solution (coating film component) | 24.3 g |
| • Mannitol | 16.2 g |
| • Light anhydrous silicic acid (water-insoluble particles) | 4.0 g |
| • Purified water | 64.3 g |

### [Overlying layer 2A spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 146.3 g |
| • Mannitol | 14.6 g |
| • Talc (water-insoluble particles) | 5.9 g |
| • Purified water | 125.9 g |

400 g of the primary fine granules thus obtained were introduced into a fluidized bed granulator (type: MP-01 (SPC)), and the charge air temperature of the fluidized bed granulator was adjusted to 40°C, the air supply rate to from 0.6 m³/h to 1 m³/h, and the liquid velocity to about 4 g/min.

937.1 g (defined amount) of a bitterness-masking layer 2A spray liquid and 220.4 g (defined amount) of an overcoat layer 2A spray liquid, all of which had compositions as described below and had been prepared in advance, were spray-coated successively in this order on the primary fine granules, and then drying was performed for 30 minutes by setting the charge air temperature of the fluidized bed granulator to 80°C. Thus, fine granules of Production Example 2 [layer configuration: core particle / active pharmaceutical ingredient layer 2A / intermediate layer 2A (underlying layer 2A / overlying layer 2A) / bitterness-masking layer 2A / overcoat layer 2A] were obtained.

### [Bitterness-masking layer 2A spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 337.0 g |
| • Aminoalkyl methacrylate copolymer E (trade name: EUDRAGIT (registered trademark) EPO, gastric polymer, Evonik Industries AG) | 10.27 g |
| • Triethyl citrate | 10.1 g |
| • Sodium lauryl sulfate | 1.03 g |
| • Stearic acid | 1.54 g |
| • Talc | 55.7 g |
| • Purified water | 521.5 g |

### [Overcoat layer 2A spray liquid]

| | |
|---|---|
| • Mannitol | 140.0 g |
| • Purified water | 860.0 g |

### <Production Example 3>

400 g of crystalline cellulose (granules) (trade name: CELPHERE (registered trademark) CP-203) as core particles was introduced into a fluidized bed granulator (type: MP-01 (SPC)), and the charge air temperature of the fluidized bed granulator was adjusted to 60°C, the air supply rate to from 0.6 m³/h to 1 m³/h, and the liquid velocity to about 2 g/min.

The entire amount of an active pharmaceutical ingredient layer 3A spray liquid, the entire amount of an underlying layer 3A spray liquid, and the entire amount of an overlying layer 3A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 3A / intermediate layer 3A (underlying layer 3A / overlying layer 3A)] were obtained.

### [Active pharmaceutical ingredient layer 3A spray liquid]

| | |
|---|---|
| • Butylscopolamine bromide (active ingredient, antispasmodic agent) | 107.98 g |
| • Hydroxypropyl cellulose (type SSL) 10 mass% aqueous solution | 162.0 g |
| • Light anhydrous silicic acid (water-insoluble particles) | 27.0 g |
| • Purified water | 422.9 g |

### [Underlying layer 3A spray liquid]

| | |
|---|---|
| • Hydroxypropyl cellulose (type SSL) 10 mass% aqueous solution (coating film component) | 24.3 g |
| • Mannitol | 16.2 g |
| • Light anhydrous silicic acid (water-insoluble particles) | 4.0 g |
| • Purified water | 64.3 g |

### [Overlying layer 3A spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 146.3 g |
| • Mannitol | 14.6 g |
| • Talc (water-insoluble particles) | 5.9 g |
| • Purified water | 125.9 g |

400 g of the primary fine granules thus obtained were introduced into a fluidized bed granulator (type: MP-01 (SPC)), and the charge air temperature of the fluidized bed granulator was adjusted to 40°C, the air supply rate to from 0.6 m³/h to 1 m³/h, and the liquid velocity to about 4 g/min.

592.6 g (defined amount) of a bitterness-masking layer 3A spray liquid and 244.4 g (defined amount) of an overcoat layer 3A spray liquid, all of which had compositions as described below and had been prepared in advance, were spray-coated successively in this order on the primary fine granules, and then drying was performed for 30 minutes by setting the charge air temperature of the fluidized bed granulator to 80°C. Thus, fine granules of Production Example 3 [layer configuration: core particle / active pharmaceutical ingredient layer 3A / intermediate layer 3A (underlying layer 3A / overlying layer 3A) / bitterness-masking layer 3A / overcoat layer 3A] were obtained.

### [Bitterness-masking layer 3A spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 592.6 g |
| • Triethyl citrate | 17.8 g |
| • Talc | 88.9 g |
| • Purified water | 723.0 g |

### [Overcoat layer 3A spray liquid]

| | |
|---|---|
| • Mannitol | 140.0 g |
| • Purified water | 860.0 g |

### <Production Example 4>

400 g of crystalline cellulose (granules) (trade name: CELPHERE (registered trademark) CP-203) as core particles was introduced into a fluidized bed granulator (type: MP-01 (SPC)), and the charge air temperature of the fluidized bed granulator was adjusted to 60°C, the air supply rate to from 0.6 m³/h to 1 m³/h, and the liquid velocity to about 2 g/min.

The entire amount of an active pharmaceutical ingredient layer 4A spray liquid and the entire amount of an underlying layer 4A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 4A / intermediate layer 4A (underlying layer 4A)] were obtained.

### [Active pharmaceutical ingredient layer 4A spray liquid]

| | |
|---|---|
| • Butylscopolamine bromide (active ingredient, antispasmodic agent) | 107.98 g |
| • Hydroxypropyl cellulose (type SSL) 10 mass% aqueous solution | 162.0 g |
| • Light anhydrous silicic acid (water-insoluble particles) | 27.0 g |
| • Purified water | 422.9 g |

### [Underlying layer 4A spray liquid]

| | |
|---|---|
| • Hydroxypropyl cellulose (type SSL) 10 mass% aqueous solution (coating film component) | 24.3 g |
| • Mannitol | 16.2 g |
| • Light anhydrous silicic acid (water-insoluble particles) | 4.0 g |
| • Purified water | 64.3 g |

12 g of the primary fine granules thus obtained were introduced into a micro fluidized bed granulating/coating apparatus (type: MICRO FLUID BED), and the charge air temperature of the apparatus was adjusted to 40°C, the air supply rate to from 10 L/min to 28 L/min, and the liquid velocity to about 0.3 g/min.

The entire amount of an overlying layer 4A spray liquid that had a composition as described below and had been prepared in advance, was spray-coated on the primary fine granules, and then drying was performed for 30 minutes by setting the charge air temperature of the apparatus to 80°C. Thus, secondary fine granules [layer configuration: core particle / active pharmaceutical ingredient layer 4A / intermediate layer 4A (underlying layer 4A / overlying layer 4A)] were obtained.

### [Overlying layer 4A spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 10.5 g |
| • Mannitol | 1.0 g |
| • Talc (water-insoluble particles) | 0.4 g |
| • Purified water | 9.0 g |

12 g of the secondary fine granules thus obtained were introduced into a micro fluidized bed granulating/coating apparatus (type: MICRO FLUID BED), and the charge air temperature of the apparatus was adjusted to 40°C, the air supply rate to from 28 L/min to 30 L/min, and the liquid velocity to about 0.3 g/min.

24.9 g (defined amount) of a bitterness-masking layer 4A spray liquid and 6.1 g (defined amount) of an overcoat layer 4A spray liquid, all of which had compositions as described below and had been prepared in advance, were spray-coated successively in this order on the secondary fine granules, and then drying was performed for 30 minutes by setting the charge air temperature of the apparatus to 80°C. Thus, fine granules of Production Example 4 [layer configuration: core particle / active pharmaceutical ingredient layer 4A / intermediate layer 4A (underlying layer 4A / overlying layer 4A) / bitterness-masking layer 4A / overcoat layer 4A] were obtained.

### [Bitterness-masking layer 4A spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 10.4 g |
| • Triethyl citrate | 0.3 g |
| • Talc | 1.6 g |
| • Purified water | 12.7 g |

### [Overcoat layer 4A spray liquid]

| | |
|---|---|
| • Mannitol | 140.0 g |
| • Purified water | 860.0 g |

### <Production Example 5>

400 g of crystalline cellulose (granules) (trade name: CELPHERE (registered trademark) CP-203) as core particles was introduced into a fluidized bed granulator (type: MP-01 (SPC)), and the charge air temperature of the fluidized bed granulator was adjusted to 60°C, the air supply rate to from 0.6 m³/h to 1 m³/h, and the liquid velocity to about 2 g/min.

The entire amount of an active pharmaceutical ingredient layer 5A spray liquid and the entire amount of an underlying layer 5A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 5A / intermediate layer 5A (underlying layer 5A)] were obtained.

### [Active pharmaceutical ingredient layer 5A spray liquid]

| | |
|---|---|
| • Butylscopolamine bromide (active ingredient, antispasmodic agent) | 107.98 g |
| • Hydroxypropyl cellulose (type SSL) 10 mass% aqueous solution | 162.0 g |
| • Light anhydrous silicic acid (water-insoluble particles) | 27.0 g |
| • Purified water | 422.9 g |

### [Underlying layer 5A spray liquid]

| | |
|---|---|
| • Hydroxypropyl cellulose (type SSL) 10 mass% aqueous solution (coating film component) | 24.3 g |
| • Mannitol | 16.2 g |
| • Light anhydrous silicic acid (water-insoluble particles) | 4.0 g |
| • Purified water | 64.3 g |

12 g of the primary fine granules thus obtained were introduced into a micro fluidized bed granulating/coating apparatus (type: MICRO FLUID BED), and the charge air temperature of the apparatus was adjusted to 40°C, the air supply rate to from 10 L/min to 28 L/min, and the liquid velocity to about 0.3 g/min.

The entire amount of an overlying layer 5A spray liquid that had a composition as described below and had been prepared in advance, was spray-coated on the primary fine granules, and then drying was performed for 30 minutes by setting the charge air temperature of the apparatus to 80°C. Thus, secondary fine granules [layer configuration: core particle / active pharmaceutical ingredient layer 5A / intermediate layer 5A (underlying layer 5A / overlying layer 5A)] were obtained.

### [Overlying layer 5A spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 5.1 g |
| • Mannitol | 0.5 g |
| • Talc (water-insoluble particles) | 0.2 g |
| • Purified water | 4.4 g |

12 g of the secondary fine granules thus obtained were introduced into a micro fluidized bed granulating/coating apparatus (type: MICRO FLUID BED), and the charge air temperature of the apparatus was adjusted to 40°C, the air supply rate to from 28 L/min to 30 L/min, and the liquid velocity to about 0.3 g/min.

30.1 g (defined amount) of a bitterness-masking layer 5A spray liquid and 7.3 g (defined amount) of an overcoat layer 5A spray liquid, all of which had compositions as described below and had been prepared in advance, were spray-coated successively in this order on the secondary fine granules, and then drying was performed for 30 minutes by setting the charge air temperature of the apparatus to 80°C. Thus, fine granules of Production Example 5 [layer configuration: core particle / active pharmaceutical ingredient layer 5A / intermediate layer 5A (underlying layer 5A / overlying layer 5A) / bitterness-masking layer 5A / overcoat layer 5A] were obtained.

### [Bitterness-masking layer 5A spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 12.5 g |
| • Triethyl citrate | 0.4 g |

| | |
|---|---|
| • Talc | 1.9 g |
| • Purified water | 15.3 g |

### [Overcoat layer 5A spray liquid]

| | |
|---|---|
| • Mannitol | 140.0 g |
| • Purified water | 860.0 g |

### <Production Example 6>

400 g of crystalline cellulose (granules) (trade name: CELPHERE (registered trademark) CP-203) as core particles was introduced into a fluidized bed granulator (type: MP-01 (SPC)), and the charge air temperature of the fluidized bed granulator was adjusted to 60°C, the air supply rate to from 0.6 m³/h to 1 m³/h, and the liquid velocity to about 2 g/min.

The entire amount of an active pharmaceutical ingredient layer 6A spray liquid, the entire amount of an underlying layer 6A spray liquid, and the entire amount of an overlying layer 6A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 6A / intermediate layer 6A (underlying layer 6A / overlying layer 6A)] were obtained.

### [Active pharmaceutical ingredient layer 6A spray liquid]

| | |
|---|---|
| • Butylscopolamine bromide (active ingredient, antispasmodic agent) | 107.98 g |
| • Hydroxypropyl cellulose (type SSL) 10 mass% aqueous solution | 162.0 g |
| • Light anhydrous silicic acid (water-insoluble particles) | 27.0 g |
| • Purified water | 422.9 g |

### [Underlying layer 6A spray liquid]

| | |
|---|---|
| • Hydroxypropyl cellulose (type SSL) 10 mass% aqueous solution (coating film component) | 24.3 g |
| • Mannitol | 16.2 g |
| • Light anhydrous silicic acid (water-insoluble particles) | 4.0 g |
| • Purified water | 64.3 g |

### [Overlying layer 6A spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 146.3 g |
| • Mannitol | 14.6 g |
| • Talc (water-insoluble particles) | 5.9 g |
| • Purified water | 125.9 g |

400 g of the primary fine granules thus obtained were introduced into a fluidized bed granulator (type: MP-01 (SPC)), and the charge air temperature of the fluidized bed granulator was adjusted to 40°C, the air supply rate to from 0.6 m³/h to 1 m³/h, and the liquid velocity to about 4 g/min.

1422.2 g (defined amount) of a bitterness-masking layer 6A spray liquid and 244.4 g (defined amount) of an overcoat layer 6A spray liquid, all of which had compositions as described below and had been prepared in advance, were spray-coated successively in this order on the primary fine granules, and then drying was performed for 30 minutes by setting the charge air temperature of the fluidized bed granulator to 80°C. Thus, fine granules of Production Example 6 [layer configuration: core particle / active pharmaceutical ingredient layer 6A / intermediate layer 6A (underlying layer 6A / overlying layer 6A) / bitterness-masking layer 6A / overcoat layer 6A] were obtained.

### [Bitterness-masking layer 6A spray liquid]

| | |
|---|---|
| • Aminoalkyl methacrylate copolymer RS dispersion liquid (water-insoluble polymer) (trade name: EUDRAGIT (registered trademark) RL30D, 30 mass% aqueous dispersion liquid, Evonik Industries AG) | 592.6 g |
| • Triethyl citrate | 17.8 g |
| • Talc | 88.9 g |
| • Purified water | 1422.2 g |

### [Overcoat layer 6A spray liquid]

| | |
|---|---|
| • Mannitol | 140.0 g |
| • Purified water | 860.0 g |

### <Production Example 7>

15 g of crystalline cellulose (granules) (trade name: CELPHERE (registered trademark) CP-203) as core particles was introduced into a micro fluidized bed granulating/coating apparatus (type: MICRO FLUID BED), and the charge air temperature of the apparatus was adjusted to 60°C, the air supply rate to from 28 L/min to 30 L/min, and the liquid velocity to about 0.3 g/min.

The entire amount of an active pharmaceutical ingredient layer 7A spray liquid and the entire amount of an overlying layer 7A spray liquid, all of which had compositions as described below and had been prepared in advance, were spray-coated in this order on the core particles, and then drying was performed for 30 minutes by setting the charge air temperature of the apparatus to 80°C. Thus, primary fine granules [layer configuration: core particle / active pharmaceutical ingredient layer 7A / intermediate layer 7A (overlying layer 7A)] were obtained.

### [Active pharmaceutical ingredient layer 7A spray liquid]

| | |
|---|---|
| • Butylscopolamine bromide (active ingredient, antispasmodic agent) | 2.46 g |
| • Hydroxypropyl cellulose (type SSL) 10 mass% aqueous solution | 3.70 g |
| • Light anhydrous silicic acid (water-insoluble particles) | 0.62 g |
| • Purified water | 9.65 g |

### [Overlying layer 7A spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 8.38 g |
| • Mannitol | 0.84 g |
| • Talc (water-insoluble particles) | 0.34 g |
| • Purified water | 7.21 g |

12 g of the primary fine granules thus obtained were introduced into a micro fluidized bed granulating/coating apparatus (type: MICRO FLUID BED), and the charge air temperature of the apparatus was adjusted to 40°C, the air supply rate to from 28 L/min to 30 L/min, and the liquid velocity to about 0.3 g/min.

27.9 g (defined amount) of a bitterness-masking layer 7A spray liquid and 4.7 g (defined amount) of an overcoat layer 7A spray liquid, all of which had compositions as described below and had been prepared in advance, were spray-coated successively in this order on the primary fine granules, and then drying was performed for 30 minutes by setting the charge air temperature of the apparatus to 80°C. Thus, fine granules of Production Example 7 [layer configuration: core particle / active pharmaceutical ingredient layer 7A / intermediate layer 7A (overlying layer 7A) / bitterness-masking layer 7A / overcoat layer 7A] were obtained.

[Bitterness-masking layer 7A spray liquid]

| | |
|---|---|
| • Aminoalkyl methacrylate copolymer RS dispersion liquid (water-insoluble polymer) (trade name: EUDRAGIT (registered trademark) RL30D, 30 mass% aqueous dispersion liquid, Evonik Industries AG) | 11.6 g |
| • Triethyl citrate | 0.35 g |
| • Talc | 1.74 g |
| • Purified water | 7.79 g |

### [Overcoat layer 7A spray liquid]

| | |
|---|---|
| • Mannitol | 140.0 g |
| • Purified water | 860.0 g |

### <Production Example 8>

400 g of crystalline cellulose (granules) (trade name: CELPHERE (registered trademark) CP-102) as core particles was introduced into a fluidized bed granulator (type: MP-01 (SPC)), and the charge air temperature of the fluidized bed granulator was adjusted to 80°C, the air supply rate to from 0.6 m³/h to 1 m³/h, and the liquid velocity to about 4 g/min.

The entire amount of an active pharmaceutical ingredient layer 8A spray liquid, the entire amount of an underlying layer 8A spray liquid, and the entire amount of an overlying layer 8A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 8A / intermediate layer 8A (underlying layer 8A / overlying layer 8A)] were obtained.

### [Active pharmaceutical ingredient layer 8A spray liquid]

| | |
|---|---|
| • Butylscopolamine bromide (active ingredient, antispasmodic agent) | 107.5 g |
| • Hydroxypropyl cellulose (type SSL) 10 mass% aqueous solution | 161.3 g |
| • Talc (water-insoluble particles) | 26.9 g |
| • Purified water | 421.2 g |

### [Underlying layer 8A spray liquid]

| | |
|---|---|
| • Hydroxypropyl cellulose (type SSL) 10 mass% aqueous solution (coating film component) | 36.5 g |
| • Mannitol | 24.3 g |
| • Talc (water-insoluble particles) | 6.1 g |
| • Purified water | 95.2 g |

### [Overlying layer 8A spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 223.6 g |
| • Mannitol | 22.4 g |
| • Talc (water-insoluble particles) | 8.9 g |
| • Purified water | 192.3 g |

400 g of the primary fine granules thus obtained were introduced into a fluidized bed granulator (type: MP-01 (SPC)), and the charge air temperature of the fluidized bed granulator was adjusted to 40°C, the air supply rate to from 0.6 m³/h to 1 m³/h, and the liquid velocity to about 4 g/min.

2278 g (defined amount) of a bitterness-masking layer 8A spray liquid and 420 g (defined amount) of an overcoat layer 8A spray liquid, all of which had compositions as described below and had been prepared in advance, were spray-coated successively in this order on the primary fine granules, and then drying was performed for 30 minutes by setting the charge air temperature of the fluidized bed granulator to 80°C. Thus, fine granules of Production Example 8 [layer configuration: core particle / active pharmaceutical ingredient layer 8A / intermediate layer 8A (underlying layer 8A / overlying layer 8A) / bitterness-masking layer 8A / overcoat layer 8A] were obtained.

### [Bitterness-masking layer 8A spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 542.4 g |
| • Aminoalkyl methacrylate copolymer RS dispersion liquid (water-insoluble polymer) (trade name: EUDRAGIT (registered trademark) RL30D, 30 mass% aqueous dispersion liquid, Evonik Industries AG) | 542.4 g |
| • Ethyl acrylate/methyl methacrylate copolymer dispersion liquid (water-insoluble polymer) (trade name: EUDRAGIT (registered trademark) NE30D, 30 mass% aqueous dispersion liquid, Evonik Industries AG) | 271.2 g |
| • Triethyl citrate | 32.5 g |
| • Talc | 244.1 g |
| • Purified water | 1784.5 g |

### [Overcoat layer 8A spray liquid]

| | |
|---|---|
| • Mannitol | 140.0 g |
| • Purified water | 860.0 g |

### <Comparative Production Example 1>

15 g of crystalline cellulose (granules) (trade name: CELPHERE (registered trademark) CP-203) as core particles was introduced into a micro fluidized bed granulating/coating apparatus (type: MICRO FLUID BED), and the charge air temperature of the apparatus was adjusted to 60°C, the air supply rate to from 28 L/min to 30 L/min, and the liquid velocity to about 0.3 g/min.

The entire amount of an active pharmaceutical ingredient layer 1B spray liquid, the entire amount of an underlying layer 1B spray liquid, and the entire amount of an overlying layer 1B spray liquid, all of which had compositions as described below and had been prepared in advance, were spray-coated in this order on the core particles, and then drying was performed for 30 minutes by setting the charge air temperature of the apparatus to 80°C. Thus, primary fine granules [layer configuration: core particle / active pharmaceutical ingredient layer 1B / intermediate layer 1B (underlying layer 1B / overlying layer 1B)] were obtained.

### [Active pharmaceutical ingredient layer 1B spray liquid]

| | |
|---|---|
| • Butylscopolamine bromide (active ingredient, antispasmodic agent) | 2.46 g |
| • Hydroxypropyl cellulose (type SSL) 10 mass% aqueous solution | 3.7 g |
| • Light anhydrous silicic acid (water-insoluble particles) | 0.62 g |
| • Purified water | 9.6 g |

### [Underlying layer 1B spray liquid]

| | |
|---|---|
| • Hydroxypropyl cellulose (type SSL) 10 mass% aqueous solution (coating film component) | 0.84 g |
| • Mannitol | 0.56 g |
| • Light anhydrous silicic acid (water-insoluble particles) | 0.14 g |
| • Purified water | 2.2 g |

### [Overlying layer 1B spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 5.1 g |
| • Mannitol | 0.5 g |
| • Talc (water-insoluble particles) | 0.2 g |
| • Purified water | 4.4 g |

12 g of the primary fine granules thus obtained were introduced into a micro fluidized bed granulating/coating apparatus (type: MICRO FLUID BED), and the charge air temperature of the apparatus was adjusted to 40°C, the air supply rate to from 28 L/min to 30 L/min, and the liquid velocity to about 0.3 g/min.

27.9 g (defined amount) of a bitterness-masking layer 1B spray liquid and 6.9 g (defined amount) of an overcoat layer 1B spray liquid, all of which had compositions as described below and had been prepared in advance, were spray-coated successively in this order on the primary fine granules, and then drying was performed for 30 minutes by setting the charge air temperature of the apparatus to 80°C. Thus, fine granules of Comparative Production Example 1 [layer configuration: core particle / active pharmaceutical ingredient layer 1B / intermediate layer 1B (underlying layer 1B / overlying layer 1B) / bitterness-masking layer 1B / overcoat layer 1B] were obtained.

### [Bitterness-masking layer 1B spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 13.5 g |
| • Triethyl citrate | 0.40 g |
| • Glycerin monostearate (GMS: Glyceryl monostearate) | 0.81 g |
| • Polysorbate | 0.32 g |
| • Purified water | 12.88 g |

### [Overcoat layer 1B spray liquid]

| | |
|---|---|
| • Mannitol | 140.0 g |
| • Purified water | 860.0 g |

### <Comparative Production Example 2>

400 g of crystalline cellulose (granules) (trade name: CELPHERE (registered trademark) CP-203) as core particles was introduced into a fluidized bed granulator (type: MP-01 (SPC)), and the charge air temperature of the fluidized bed granulator was adjusted to 60°C, the air supply rate to from 0.6 m³/h to 1 m³/h, and the liquid velocity to about 2 g/min.

The entire amount of an active pharmaceutical ingredient layer 2B spray liquid and the entire amount of an underlying layer 2B spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 2B / intermediate layer 2B (underlying layer 2B)] were obtained.

### [Active pharmaceutical ingredient layer 2B spray liquid]

| | |
|---|---|
| • Butylscopolamine bromide (active ingredient, antispasmodic agent) | 107.98 g |
| • Hydroxypropyl cellulose (type SSL) 10 mass% aqueous solution | 162.0 g |
| • Light anhydrous silicic acid (water-insoluble particles) | 27.0 g |
| • Purified water | 422.9 g |

### [Underlying layer 2B spray liquid]

| | |
|---|---|
| • Hydroxypropyl cellulose (type SSL) 10 mass% aqueous solution (coating film component) | 24.3 g |
| • Mannitol | 16.2 g |
| • Light anhydrous silicic acid (water-insoluble particles) | 4.0 g |
| • Purified water | 64.3 g |

12 g of the primary fine granules thus obtained were introduced into a micro fluidized bed granulating/coating apparatus (type: MICRO FLUID BED), and the charge air temperature of the apparatus was adjusted to 40°C, the air supply rate to from 10 L/min to 28 L/min, and the liquid velocity to about 0.3 g/min.

The entire amount of an overlying layer 2B spray liquid that had a composition as described below and had been prepared in advance, was spray-coated on the primary fine granules, and then drying was performed for 30 minutes by setting the charge air temperature of the apparatus to 80°C. Thus, secondary fine granules [layer configuration: core particle / active pharmaceutical ingredient layer 2B / intermediate layer 2B (underlying layer 2B / overlying layer 2B)] were obtained.

### [Overlying layer 2B spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 10.5 g |
| • Mannitol | 1.0 g |
| • Talc (water-insoluble particles) | 0.4 g |
| • Purified water | 9.0 g |

10 g of the secondary fine granules thus obtained were introduced into a micro fluidized bed granulating/coating apparatus (type: MICRO FLUID BED), and the charge air temperature of the apparatus was adjusted to 40°C, the air supply rate to from 28 L/min to 30 L/min, and the liquid velocity to about 0.3 g/min.

70.6 g (defined amount) of a bitterness-masking layer 2B spray liquid and 6.2 g (defined amount) of an overcoat layer 2B spray liquid, all of which had compositions as described below and had been prepared in advance, were spray-coated successively in this order on the secondary fine granules, and then drying was performed for 30 minutes by setting the charge air temperature of the apparatus to 80°C. Thus, fine granules of Comparative Production Example 2 [layer configuration: core particle / active pharmaceutical ingredient layer 2B / intermediate layer 2B (underlying layer 2B / overlying layer 2B) / bitterness-masking layer 2B / overcoat layer 2B] were obtained.

### [Bitterness-masking layer 2B spray liquid]

| | |
|---|---|
| • Aminoalkyl methacrylate copolymer E (trade name: EUDRAGIT (registered trademark) EPO, gastric polymer, Evonik Industries AG) | 7.1 g |
| • Sodium lauryl sulfate | 0.7 g |
| • Stearic acid | 1.9 g |
| • Glycerin monostearate (GMS: Glyceryl monostearate) | 0.4 g |
| • Polysorbate 80 | 0.1 g |
| • Purified water | 61.2 g |

### [Overcoat layer 2B spray liquid]

| | |
|---|---|
| • Mannitol | 140.0 g |
| • Purified water | 860.0 g |

### <Comparative Production Example 3>

15 g of crystalline cellulose (granules) (trade name: CELPHERE (registered trademark) CP-203) as core particles was introduced into a micro fluidized bed granulating/coating apparatus (type: MICRO FLUID BED), and the charge air temperature of the apparatus was adjusted to 60°C, the air supply rate to from 28 L/min to 30 L/min, and the liquid velocity to about 0.3 g/min.

The entire amount of an active pharmaceutical ingredient layer 3B spray liquid and the entire amount of an overlying layer 3B spray liquid, all of which had compositions as described below and had been prepared in advance, were spray-coated in this order on the core particles, and then drying was performed for 30 minutes by setting the charge air temperature of the apparatus to 80°C. Thus, primary fine granules [layer configuration: core particle / active pharmaceutical ingredient layer 3B / intermediate layer 3B (overlying layer 3B)] were obtained.

### [Active pharmaceutical ingredient layer 3B spray liquid]

| | |
|---|---|
| • Butylscopolamine bromide (active ingredient, antispasmodic agent) | 2.52 g |
| • Hydroxypropyl cellulose (type SSL) 10 mass% aqueous solution | 3.78 g |
| • Light anhydrous silicic acid (water-insoluble particles) | 0.63 g |
| • Purified water | 9.88 g |

### [Overlying layer 3B spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 12.77 g |
| • Mannitol | 1.28 g |
| • Talc (water-insoluble particles) | 0.51 g |
| • Purified water | 10.99 g |

15 g of the primary fine granules thus obtained were introduced into a micro fluidized bed granulating/coating apparatus (type: MICRO FLUID BED), and the charge air temperature of the apparatus was adjusted to 40°C, the air supply rate to from 28 L/min to 30 L/min, and the liquid velocity to about 0.3 g/min.

9.4 g (defined amount) of a bitterness-masking layer 3B spray liquid and 7.5 g (defined amount) of an overcoat layer 3B spray liquid, all of which had compositions as described below and had been prepared in advance, were spray-coated successively in this order on the primary fine granules, and then drying was performed for 30 minutes by setting the charge air temperature of the apparatus to 80°C. Thus, fine granules of Comparative Production Example 3 [layer configuration: core particle / active pharmaceutical ingredient layer 3B / intermediate layer 3B (overlying layer 3B) / bitterness-masking layer 3B / overcoat layer 3B] were obtained.

### [Bitterness-masking layer 3B spray liquid]

| | |
|---|---|
| • Aminoalkyl methacrylate copolymer RS dispersion liquid (water-insoluble polymer) (trade name: EUDRAGIT (registered trademark) RL30D, 30 mass% aqueous dispersion liquid, Evonik Industries AG) | 4.20 g |
| • Triethyl citrate | 0.25 g |
| • Polysorbate 80 | 0.10 g |
| • Glycerin monostearate (GMS: Glyceryl monostearate) | 0.25 g |
| • Purified water | 2.51 g |

### [Overcoat layer 3B spray liquid]

| | |
|---|---|
| • Mannitol | 140.0 g |
| • Purified water | 860.0 g |

### <Comparative Production Example 4>

15 g of crystalline cellulose (granules) (trade name: CELPHERE (registered trademark) CP-203) as core particles was introduced into a micro fluidized bed granulating/coating apparatus (type: MICRO FLUID BED), and the charge air temperature of the apparatus was adjusted to 60°C, the air supply rate to from 28 L/min to 30 L/min, and the liquid velocity to about 0.3 g/min.

The entire amount of an active pharmaceutical ingredient layer 4B spray liquid that had a composition as described below and had been prepared in advance, was spray-coated on the core particles, and aggregation occurred so noticeably that fine granules could not be obtained.

### [Active pharmaceutical ingredient layer 4B spray liquid]

| | |
|---|---|
| • Butylscopolamine bromide (active ingredient, antispasmodic agent) | 2.99 g |
| • Hydroxypropyl cellulose (type SSL) 10 mass% aqueous solution | 4.88 g |
| • Purified water | 11.19 g |

### [Evaluation on bitterness concealing]

The fine granules of Production Examples 1 to 8 and Comparative Production Examples 1 to 3 were respectively measured such that 10 mg of butylscopolamine bromide would be included in a dose, and the fine granules were inserted into the oral cavities of test subjects. The time period for which bitterness was concealed (hereinafter, referred to as "bitterness masking time") was measured, and an evaluation on bitterness concealing was performed according to the following evaluation criteria. Meanwhile, in Comparative Production Example 4, fine granules could not be formed, and therefore, the evaluation on bitterness concealing was not performed.

If a sample is rated [A] or [B] of the following evaluation criteria, there is no problem in practical use.

### - Evaluation criteria -

A: The bitterness masking time is 60 seconds or more.
B: The bitterness masking time is 30 seconds or more and less than 60 seconds.
C: The bitterness masking time is less than 30 seconds.

The compositions of the spray liquids for forming the various layers of the fine granules of Production Examples 1 to 8 and Comparative Production Examples 1 to 3 are presented in Table 1.

The compositions and the evaluation results for the fine granules of Production Examples 1 to 8 and Comparative Examples 1 to 3 are presented in Table 2.

In Table 1 and Table 2, the unit "percent (%)" means "percent (%) by mass".

In Table 1 and Table 2, the symbol "-" means that there is no relevant data, and for example, in the columns for the composition, it is implied that the relevant component has not been incorporated.

**[Table 1]**

| | | | Production Example 1 | Production Example 2 | Production Example 3 | Production Example 4 | Production Example 5 | Production Example 6 | Production Example 7 | Production Example 8 | Comparative Production Example 1 | Comparative Production Example 2 | Comparative Production Example 3 |
|---|---|---|---|---|---|---|---|---|---|---|---|---|---|
| Active pharmaceutical ingredient- containing core | Core particle | Crystalline cellulose | - | - | - | - | - | - | - | - | - | - | - |
| | Active pharmaceutical ingredient layer | Butylscopolamine bromide | 15.0% | 15.0% | 15.0% | 15.0% | 15.0% | 15.0% | 15.0% | 15.0% | 15.0% | 15.0% | 15.0% |
| | | Hydroxypropyl cellulose (HPC) 10 mass% aqueous | 22.5% | 22.5% | 22.5% | 22.5% | 22.5% | 22.5% | 22.5% | 22.5% | 22.5% | 22.5% | 22.5% |
| | | Light anhydrous silicic acid | 3.8% | 3.8% | 3.8% | 3.8% | 3.8% | 3.8% | 3.8% | - | 3.8% | 3.8% | 3.8% |
| | | Talc | - | - | - | - | - | - | - | 3.8% | - | - | - |
| | | Purified water | 58.7% | 58.7% | 58.7% | 58.7% | 58.7% | 58.7% | 58.7% | 58.7% | 58.7% | 58.7% | 58.7% |
| Intermediate layer | Underlying layer | Hydroxypropyl cellulose (HPC) 10 mass% aqueous solution | 22.5% | 22.5% | 22.5% | 22.5% | 22.5% | 22.5% | - | 22.5% | 22.5% | 22.5% | - |
| | | Mannitol | 15.0% | 15.0% | 15.0% | 15.0% | 15.0% | 15.0% | - | 15.0% | 15.0% | 15.0% | - |
| | | Light anhydrous silicic acid | 3.8% | 3.8% | 3.8% | 3.8% | 3.8% | 3.8% | - | - | 3.8% | 3.8% | - |
| | | Talc | - | - | - | - | - | - | - | 3.8% | - | - | - |
| | | Purified water | 58.7% | 58.7% | 58.7% | 58.7% | 58.7% | 58.7% | - | 58.7% | 58.7% | 58.7% | - |
| | Overlying layer | Hydroxypropyl methylcellulose (HPMC) | 50.0% | 50.0% | 50.0% | 50.0% | 50.0% | 50.0% | 50.0% | 50.0% | 50.0% | 50.0% | 50.0% |
| | | Mannitol | 5.0% | 5.0% | 5.0% | 5.0% | 5.0% | 5.0% | 5.0% | 5.0% | 5.0% | 5.0% | 5.0% |
| | | Talc | 2.0% | 2.0% | 2.0% | 2.0% | 2.0% | 2.0% | 2.0% | 2.0% | 2.0% | 2.0% | 2.0% |
| | | Purified water | 43.0% | 43.0% | 43.0% | 43.0% | 43.0% | 43.0% | 43.0% | 43.0% | 43.0% | 43.0% | 43.0% |
| Bitterness-masking layer | | Aminoalkyl methacrylate polymer RS (EUDRAGIT RS30D) | 20.8% | 36.0% | 41.7% | 41.7% | 41.7% | - | - | - 15.9% | 48.3% | - | - |
| | | Aminoalkyl methacrylate polymer RS (EUDRAGIT RL30D) | 20.8% 801 | - | - | - | - | 41.7% | 41.7% | 15.9% | - | - | 45.0% |
| | | Aminoalkyl methacrylate polymer E (EUDRAGIT EPO) | - | 1.1% | - | - | - | - | - | - | - | 10.0% | - |
| | | Ethyl acrylate/methyl methacrylate copolymer (EUDRAGIT NE30D) | - | - | - | - | - | - | - | 7.9% | - | - | - |
| | | Triethyl citrate | 1.3% | 1.1% | 1.3% | 1.3% | 1.3% | 1.3% | 1.3% | 1.0% | 1.4% | - | 2.7% |
| | | Talc | 6.3% | 5.9% | 6.3% | 6.3% | 6.3% | 6.3% | 6.3% | 7.1% | - | - | - |
| | | Sodium lauryl sulfate | - | 0.1% | - | - | - | - | - | - | - | 1.0% | - |
| | | Stearic acid | - | 0.2% | - | - | - | - | - | - | - | 1.5% | - |
| | | GMS | - | - | - | - | - | - | - | - | 2.9% | 0.5% | 2.7% |
| | | Polysorbate 80 | - | - | - | - | - | - | - | - | 1.1% | 0.2% | 1.1% |
| | | Purified water | 50.8% | 55.6% | 50.7% | 50.7% | 50.7% | 50.7% | 50.7% | 52.2% | 46.3% | 86.8% | 48.5% |
| Overcoat layer | | Mannitol | 14.0% | 14.0% | 14.0% | 14.0% | 14.0% | 14.0% | 14.0% | 14.0% | 14.0% | 14.0% | 14.0% |
| | | Purified water | 86.0% | 86.0% | 86.0% | 86.0% | 86.0% | 86.0% | 86.0% | 86.0% | 86.0% | 86.0% | 86.0% |

**[Table 2]**

| | | | Production Example 1 | Production Example 2 | Production Example 3 | Production Example 4 | Production Example 5 | Production Example 6 | Production Example 7 | Production Example 8 | Comparative Production Example 1 | Comparative Production Example 2 | Comparative Production Example 3 |
|---|---|---|---|---|---|---|---|---|---|---|---|---|---|
| Active pharmaceutical ingredient- containing core | Core particle | Crystalline cellulose | 36.6% | 35.4% | 36.6% | 36.6% | 39.7% | 36.6% | 47.5% | 27.4% | 47.5% | 26.9% | 55.2% |
| | Active pharmaceutical ingredient layer | Butylscopolamine bromide | 9.9% | 9.6% | 99% | 9.9% | 10.7% | 9.9% | 7.8% | 7.4% | 7.8% | 7.3% | 9.3% |
| | | Hydroxypropyl cellulose (HPC) | 1.5% | 1.4% | 1.5% | 1.5% | 1.6% | 1.5% | 1.2% | 1.1% | 1.2% | 1.1% | 1.4% |
| | | Talc | - | - | - | - | - | - | - | 1.8% | - | - | - |
| | | Light anhydrous silicic acid | 2.5% | 2.4% | 2.5% | 2.5% | 2.7% | 2.5% | 1.9% | - | 1.9% | 1.8% | 2.3% |
| Intermediate layer | Underlying layer | Film thickness (µm) | 2 | 2 | 2 | 2 | 2 | 2 | 0 | 2 | 2 | 2 | 0 |
| | | Hydroxypropyl cellulose (HPC) | 0.2% | 0.2% | 0.2% | 0.2% | 0.2% | 0.2% | - | 0.3% | 0.3% | 0.2% | - |
| | | Mannitol | 1.5% | 1.4% | 1.5% | 1.5% | 1.6% | 1.5% | - | 1.7% | 1.8% | 1.1% | - |
| | | Talc | - | - | - | - | - | - | - | 0.4% | - | - | - |
| | | Light anhydrous silicic acid | 0.4% | 0.4% | 0.4% | 0.4% | 0.4% | 0.4% | - | - | 0.4% | 0.3% | - |
| | Overlying layer | Film thickness (µm) | 3 | 3 | 3 | 13 | 8 | 3 | 5 | 3 | 3 | 13 | 5 |
| | | Hydroxypropyl methylcellulose (HPMC) | 1.3% | 2.6% | 1.3% | 6.1% | 4.0% | 1.3% | 2.7% | 1.5% | 1.6% | 4.6% | 4.7% |
| | | Mannitol | 1.3% | 2.6% | 1.3% | 6.1% | 4.0% | 1.3% | 2.7% | 1.5% | 1.6% | 4.6% | 4.7% |
| | | Talc | 0.5% | 1.0% | 0.5% | 2.5% | 1.6% | 0.5% | 1.1% | 0.6% | 0.6% | 1.8% | 1.9% |
| Bitterness-masking layer | | Aminoalkyl methacrylate polymer RS (EUDRAGIT RS30D) | 12.3% | 21.6% | 24.6% | 17.5% | 17.9% | - | - | 11.9% | 21.8% | - | - |
| | | Aminoalkyl methacrylate polymer RS (EUDRAGIT RL30D) | 12.3% | - | - | - | - | 24.6% | 18.8% | 11.9% | - | - | 8.4% |
| | | Aminoalkyl methacrylate polymer E (EUDRAGIT EPO) | - | 2.2% | - | - | - | - | - | - | - | 35.0% | - |
| | | Ethyl acrylate/methyl methacrylate copolymer (EUDRAGIT NE30D) | - | - | - | - | - | - | - | 5.9% | - | - | - |
| | | Triethyl citrate | 2.5% | 2.2% | 2.5% | 1.7% | 1.8% | 2.5% | 1.9% | 2.4% | 2.2% | - | 1.7% |
| | | Talc | 12.4% | 1.9% | 12.4% | 8.7% | 8.9% | 12.4% | 9.4% | 17.8% | - | - | - |
| | | Sodium lauryl sulfate | - | 0.2% | - | - | - | - | - | - | - | 3.5% | - |
| | | Stearic acid | - | 0.3% | - | - | - | - | - | - | - | 5.2% | - |
| | | GMS | - | - | - | - | - | - | - | - | 4.4% | 1.7% | 1.7% |
| | | Polysorbate 80 | - | - | - | - | - | - | - | - | 1.7% | 0.7% | 0.7% |
| Overcoat layer | | Mannitol | 4.8% | 4.6% | 4.8% | 4.8% | 4.9% | 4.8% | 5.0% | 6.4% | 5.2% | 4.2% | 8.0% |
| Evaluation on bitterness concealing | | | A | A | A | B | A | B | B | A | C | C | C |

As shown in Table 2, in the fine granules of Production Examples 1 to 8, the bitter taste attributed to butylscopolamine bromide was sufficiently concealed.

On the other hand, in the fine granules of Comparative Production Examples 1 to 3 that did not have a bitterness-masking layer including talc and a water-insoluble polymer, the bitter taste attributed to butylscopolamine bromide could not be sufficiently concealed.

The fine granules of Production Example 3 and Production Example 5, in which the thickness of the intermediate layer was 10 µm or less, had an excellent effect of concealing the bitter taste attributed to butylscopolamine bromide, compared to the fine granules of Production Example 4, in which the thickness was more than 10 µm.

The fine granules of Production Example 3 that included EUDRAGIT (registered trademark) RS30D as a water-insoluble polymer in the bitterness-masking layer had an excellent effect of concealing the bitter taste attributed to butylscopolamine bromide, compared to the fine granules of Production Example 6 that included EUDRAGIT (registered trademark) RL30D.

### [Evaluation of orally disintegrating tablet]

### <<Example 1>>

The fine granules of Production Example 1 and excipient components were mixed at the ratio of the following Formulation (1), and a tableting powder (mixed powder) was obtained. The fine granules were weighed such that butylscopolamine bromide would be included in an amount of 10 mg, and were mixed.

A defined amount of the tableting powder (mixed powder) thus obtained was measured and was subjected to compression molding at a speed of rotation of 20 rpm and a tableting pressure of about 5 kN, using a rotary tableting machine (product name: HT-AP-SS, Hata Tekkosho Co., Ltd.) and using a pounder having an angular-cornered R face with 8 mmφ. Thus, an orally disintegrating tablet (tablet preparation) of Example 1 was obtained.

### [Formulation (1)]

| | |
|---|---|
| • Fine granules | 50.0% by mass |
| • Mannitol/corn starch granulated material | 40.2% by mass |
| • Ethyl cellulose | 5.0% by mass |
| • Crospovidone | 3.0% by mass |
| • Aspartame | 1.0% by mass |
| • STRAWBERRY MICRON | 0.1% by mass |
| • Sodium stearyl fumarate | 0.7% by mass |

### <<Examples 2 to 8>>

The fine granules of Production Examples 2 to 8 and excipient components were respectively mixed at the ratio of the above-described Formulation (1), and tableting powders (mixed powders) were obtained.

Production was carried out in the same manner as in Example 1 using the tableting powders (mixed powders) thus obtained, and orally disintegrating tablets (tablet preparations) of Examples 2 to 8 were obtained.

The orally disintegrating tablets of Examples 1 to 8 thus obtained were subjected to an evaluation on bitterness concealing according to the evaluation criteria described above. As a result, for all of the orally disintegrating tablets, the same results as the evaluation results for the fine granules shown in Table 2 were obtained, and the bitter taste attributed to butylscopolamine bromide was sufficiently concealed.

Furthermore, the orally disintegrating tablet of Example 3 that included the fine granules of Production Example 3, and the orally disintegrating tablet of Example 5 that included the fine granules of Production Example 5 had an excellent effect of concealing the bitter taste attributed to butylscopolamine bromide, compared to the orally disintegrating tablet of Example 4 that included the fine granules of Production Example 4.

### <<Exaniple 9>>

The fine granules of Production Example 1 and excipient components were mixed at the ratio of the following Formulation (2), and a tableting powder (mixed powder) was obtained.

A defined amount of the tableting powder (mixed powder) thus obtained was measured and was subjected to compression molding at a speed of rotation of 20 rpm and a tableting pressure of about 5 kN, using a rotary tableting machine (product name: HT-AP-SS, Hata Tekkosho Co., Ltd.) and using a pounder having an angular-cornered R face with 9 mmφ. Thus, an orally disintegrating tablet (tablet preparation) of Example 9 was obtained.

### [Formulation (2)]

| | |
|---|---|
| • Fine granules | 50.0% by mass |
| • Mannitol/corn starch granulated material | 39.2% by mass |
| • Ethyl cellulose | 5.0% by mass |
| • Crospovidone | 3.0% by mass |
| • Aspartame | 1.0% by mass |
| • Sodium lauryl sulfate | 1.0% by mass |
| • STRAWBERRY MICRON | 0.1% by mass |
| • Sodium stearyl fumarate | 0.7% by mass |

### «Evaluation on bitterness concealing»

The orally disintegrating tablets of Example 1 and Example 9 thus obtained were inserted into the oral cavities of test subjects. An evaluation was performed according to the following evaluation criteria, on whether the bitter taste attributed to butylscopolamine bromide remained in the oral cavity 5 minutes after insertion. The results are presented in Table 3. In Table 3, the unit "percent (%)" means "percent (%) by mass", and the symbol "-" means that the relevant component has not been incorporated.

### - Evaluation criteria -

0: The bitter taste does not remain 5 minutes after insertion.
1: The bitter taste slightly remains 5 minutes after insertion.

**[Table 3]**

| | Example 9 | Example 1 |
|---|---|---|
| Fine granules | 50.0% | 50.0% |
| Mannitol/corn starch granulated material | 39.2% | 40.2% |
| Ethyl cellulose | 5.0% | 5.0% |
| Crospovidone | 3.0% | 3.0% |
| Aspartame | 1.0% | 1.0% |
| Sodium lauryl sulfate | 1.0% | - |
| STRAWBERRY MICRON | 0.1% | 0.1% |
| Sodium stearyl fumarate | 0.7% | 0.7% |
| Evaluation on bitterness concealing (bitterness remaining after 5 minutes) | 0 | 1 |

As shown in Table 3, the orally disintegrating tablet of Example 9 that included sodium lauryl sulfate (anionic substance having a pKa of 3.5 or less) on the outside of the fine granules as an excipient component, had an excellent effect of concealing the bitter taste attributed to butylscopolamine bromide, compared to the orally disintegrating tablet of Example 1 that did not include sodium lauryl sulfate. Furthermore, the orally disintegrating tablets of Example 1 and Example 9 exhibited hardness and disintegrability that were sufficient for practical use.

### [Production of fine granules]

### <Production Example 9>

400 g of D-mannitol (trade name: NONPAREIL (registered trademark)-108) as core particles was introduced into a fluidized bed granulator (type: MP-01 (SPC)), and the charge air temperature of the fluidized bed granulator was adjusted to 80°C, the air supply rate to from 0.6 m³/h to 1 m³/h, and the liquid velocity to about 4 g/min.

The entire amount of an active pharmaceutical ingredient layer 9A spray liquid, the entire amount of an underlying layer 9A spray liquid, and the entire amount of an overlying layer 9A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 9A / intermediate layer 9A (underlying layer 9A / overlying layer 9A)] were obtained.

### [Active pharmaceutical ingredient layer 9A spray liquid]

| | |
|---|---|
| • Butylscopolamine bromide (active ingredient, antispasmodic agent) | 172.2 g |
| • Hydrous silicon dioxide (water-insoluble particles) (trade name: ADSOLIDER (registered trademark) 102, Freund Corporation) | 172.2 g |
| • Purified water | 229.6 g |
| | |
| | |
| | |

### [Underlying layer 9A spray liquid]

| | |
|---|---|
| • Hydrous silicon dioxide (water-insoluble particles) (trade name: ADSOLIDER (registered trademark) 102, Freund Corporation) | 55.8 g |
| • Purified water | 209.9 g |

### [Overlying layer 9A spray liquid]

| | |
|---|---|
| • Hydroxypropyl cellulose (type SSL) 10 mass% aqueous solution (coating film component) | 243.9 g |
| • Mannitol | 24.4 g |
| • Talc (water-insoluble particles) | 9.8 g |
| • Purified water | 209.7 g |

400 g of the primary fine granules thus obtained were introduced into a fluidized bed granulator (type: MP-01 (SPC)), and the charge air temperature of the fluidized bed granulator was adjusted to 40°C, the air supply rate to from 0.6 m³/h to 1 m³/h, and the liquid velocity to about 4 g/min.

1745 g (defined amount) of a bitterness-masking layer 9A spray liquid and 309 g (defined amount) of a first overcoat layer 9A spray liquid, all of which had compositions as described below and had been prepared in advance, were spray-coated successively in this order on the primary fine granules, and then drying was performed for 30 minutes by setting the charge air temperature of the fluidized bed granulator to 80°C. Thus, secondary fine granules [layer configuration: core particle / active pharmaceutical ingredient layer 9A / intermediate layer 9A (underlying layer 9A / overlying layer 9A) / bitterness-masking layer 9A / first overcoat layer 9A] were obtained.

### [Bitterness-masking layer 9A spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 415.5 g |
| • Aminoalkyl methacrylate copolymer RS dispersion liquid (water-insoluble polymer) (trade name: EUDRAGIT (registered trademark) RL30D, 30 mass% aqueous dispersion liquid, Evonik Industries AG) 415.5 g | 415.5 g |
| • Ethyl acrylate/methyl methacrylate copolymer dispersion liquid (water-insoluble polymer) (trade name: EUDRAGIT (registered trademark) NE30D, 30 mass% aqueous dispersion liquid, Evonik Industries AG) | 207.7 g |
| • Triethyl citrate | 24.9 g |
| • Talc | 187.0 g |
| • Purified water | 1366.9 g |

### [First overcoat layer 9A spray liquid]

| | |
|---|---|
| • Mannitol | 140.0 g |
| • Purified water | 860.0 g |

130 g of the secondary fine granules thus obtained were introduced into a fluidized bed granulator (type: MP-01 (SPC)), and the charge air temperature of the fluidized bed granulator was adjusted to 60°C, the air supply rate to from 0.6 m³/h to 1 m³/h, and the liquid velocity to about 3 g/min.

47.7 g (define amount) of a second overcoat layer 9A spray liquid that had a composition as described below and had been prepared in advance was spray-coated on the secondary fine granules, and then drying was performed for 30 minutes by setting the charge air temperature of the fluidized bed granulator to 80°C. Thus, fine granules of Production Example 9 [layer configuration: core particle / active pharmaceutical ingredient layer 9A / intermediate layer 9A (underlying layer 9A / overlying layer 9A) / bitterness-masking layer 9A / first overcoat layer 9A / second overcoat layer 9A] were obtained.

### [Second overcoat layer 9A spray liquid]

| | |
|---|---|
| • Sodium lauryl sulfate | 20.0 g |
| • Purified water | 380.0 g |

### <Production Example 10>

400 g of D-mannitol (trade name: NONPAREIL (registered trademark)-108) as core particles was introduced into a fluidized bed granulator (type: MP-01 (SPC)), and the charge air temperature of the fluidized bed granulator was adjusted to 80°C, the air supply rate to from 0.6 m³/h to 1 m³/h, and the liquid velocity to about 4 g/min.

The entire amount of an active pharmaceutical ingredient layer 10A spray liquid, the entire amount of an underlying layer 10A spray liquid, and the entire amount of an overlying layer 10A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 10A / intermediate layer 10A (underlying layer 10A / overlying layer 10A)] were obtained.

### [Active pharmaceutical ingredient layer 10A spray liquid]

| | |
|---|---|
| • Butylscopolamine bromide (active ingredient, antispasmodic agent) | 172.2 g |
| • Hydrous silicon dioxide (water-insoluble particles) (trade name: ADSOLIDER (registered trademark) 102, Freund Corporation) | 172.2 g |
| • Purified water | 229.6 g |

### [Underlying layer 10A spray liquid]

| | |
|---|---|
| • Hydrous silicon dioxide (water-insoluble particles) (trade name: ADSOLIDER (registered trademark) 102, Freund Corporation) | 55.8 g |
| • Purified water | 209.9 g |

### [Overlying layer 10A spray liquid]

| | |
|---|---|
| • Hydroxypropyl cellulose (type SSL) 10 mass% aqueous solution (coating film component) | 243.9 g |
| • Mannitol | 24.4 g |
| • Talc (water-insoluble particles) | 9.8 g |
| • Purified water | 209.7 g |

400 g of the primary fine granules thus obtained were introduced into a fluidized bed granulator (type: MP-01 (SPC)), and the charge air temperature of the fluidized bed granulator was adjusted to 40°C, the air supply rate to from 0.6 m³/h to 1 m³/h, and the liquid velocity to about 4 g/min.

1745 g (defined amount) of a bitterness-masking layer 10A spray liquid and 309 g (defined amount) of an overcoat layer 10A spray liquid, all of which had compositions as described below and had been prepared in advance, were spray-coated successively in this order on the primary fine granules, and then drying was performed for 30 minutes by setting the charge air temperature of the fluidized bed granulator to 80°C. Thus, fine granules of Production Example 10 [layer configuration: core particle / active pharmaceutical ingredient layer 10A / intermediate layer 10A (underlying layer 10A / overlying layer 10A) / bitterness-masking layer 10A / overcoat layer 10A] were obtained.

### [Bitterness-masking layer 10A spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 415.5 g |
| • Aminoalkyl methacrylate copolymer RS dispersion liquid (water-insoluble (trade name: EUDRAGIT (registered trademark) RL30D, 30 mass% aqueous dispersion iquid, Evonik Industries AG) | 415.5 g |
| • Ethyl acrylate/methyl methacrylate copolymer dispersion liquid (water-insoluble polymer) (trade name: EUDRAGIT (registered trademark) NE30D, 30 mass% dispersion liquid, Evonik Industries AG) | 207.7 g |
| • Triethyl citrate | 24.9 g |
| • Talc | 187.0 g |
| • Purified water | 1366.9 g |

### [Overcoat layer 10A spray liquid]

| | |
|---|---|
| • Mannitol | 140.0 g |
| • Purified water | 860.0 g |

The compositions of the fine granules of Production Example 9 and Production Example 10 are presented in Table 4. In Table 4, the unit "percent (%)" means "percent (%) by mass", and the symbol "-" implies that the relevant component has not been incorporated.

**[Table 4]**

| | | | Production Example 9 | Production Example 10 |
|---|---|---|---|---|
| Active pharmaceutical ingredient-containing core | Core particle | Mannitol | 23.0% | 23.6% |
| | Active | Butylscopolamine bromide | 9.9% | 10.1% |
| | pharmaceutical ingredient layer | Hydrous silicon dioxide | 9.9% | 10.1% |
| Intermediate layer | Underlying | Film thickness (µm) | 2 | 2 |
| | layer | Hydrous silicon dioxide | 3.2% | 3.3% |
| | | Film thickness (µm) | 3 | 3 |
| | | Hydroxypropyl cellulose (HPC) | 1.4% | 1.4% |
| | Overlying layer | Mannitol | 1.4% | 1.4% |
| | | Talc | 0.6% | 0.6% |
| Bitterness-masking layer | | Aminoalkyl methacrylate polymer RS (EUDRAGIT RS30D) | 10.3% | 10.5% |
| | | Aminoalkyl methacrylate polymer RS (EUDRAGIT RL30D) | 10.3% | 10.5% |
| | | Ethyl acrylate/methyl methacrylate copolymer (EUDRAGIT NE30D) | 5.2% | 5.2% |
| | | Triethyl citrate | 2.1% | 2.1% |
| | | Talc | 15.5% | 15.7% |
| Overcoat layer | | Mannitol | 5.4% | 5.5% |
| | | Sodium lauryl sulfate | 1.8% | - |

### [Production of fine granules]

### <Production Example 11>

400 g of D-mannitol (trade name: NONPAREIL (registered trademark)-108) as core particles was introduced into a fluidized bed granulator (type: MP-01 (SPC)), and the charge air temperature of the fluidized bed granulator was adjusted to 80°C, the air supply rate to from 0.6 m³/h to 1 m³/h, and the liquid velocity to about 4 g/min.

The entire amount of an active pharmaceutical ingredient layer 11A spray liquid, the entire amount of an underlying layer 11A spray liquid, and the entire amount of an overlying layer 11A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 11A / intermediate layer 11A (underlying layer 11A / overlying layer 11A)] were obtained.

### [Active pharmaceutical ingredient layer 11A spray liquid]

| | |
|---|---|
| • Butylscopolamine bromide (active ingredient, antispasmodic agent) | 172.2 g |
| • Hydrous silicon dioxide (water-insoluble particles) (trade name: ADSOLIDER (registered trademark) 102, Freund Corporation) | 172.2 g |
| • Purified water | 229.6 g |

### [Underlying layer 11A spray liquid]

| | |
|---|---|
| • Hydrous silicon dioxide (water-insoluble particles) (trade name: ADSOLIDER (registered trademark) 102, Freund Corporation) | 55.8 g |
| • Purified water | 209.9 g |

### [Overlying layer 11A spray liquid]

| | |
|---|---|
| • Hydroxypropyl cellulose (type SSL) 10 mass% aqueous solution (coating film component) | 243.9 g |
| • Mannitol | 24.4 g |
| • Talc (water-insoluble particles) | 9.8 g |
| • Purified water | 209.7 g |

400 g of the primary fine granules thus obtained were introduced into a fluidized bed granulator (type: MP-01 (SPC)), and the charge air temperature of the fluidized bed granulator was adjusted to 40°C, the air supply rate to from 0.6 m³/h to 1 m³/h, and the liquid velocity to about 4 g/min.

1745 g (defined amount) of a bitterness-masking layer 11A spray liquid and 309 g (defined amount) of an overcoat layer 11A spray liquid, all of which had compositions as described below and had been prepared in advance, were spray-coated successively in this order on the primary fine granules, and then drying was performed for 30 minutes by setting the charge air temperature of the fluidized bed granulator to 80°C. Thus, fine granules of Production Example 11 [layer configuration: core particle / active pharmaceutical ingredient layer 11A / intermediate layer 11A (underlying layer 11A / overlying layer 11A) / bitterness-masking layer 11A / overcoat layer 11A] were obtained.

### [Bitterness-masking layer 11A spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 415.5 g |
| • Aminoalkyl methacrylate copolymer RS dispersion liquid (water-insoluble polymer) (trade name: EUDRAGIT (registered trademark) RL30D, 30 mass% aqueous dispersion liquid, Evonik Industries AG) | 415.5 g |
| • Ethyl acrylate/methyl methacrylate copolymer dispersion liquid (water-insoluble polymer) (trade name: EUDRAGIT (registered trademark) NE30D, 30 mass% aqueous dispersion liquid, Evonik Industries AG) | 207.7 g |
| • Triethyl citrate | 24.9 g |
| • Talc | 187.0 g |
| • Purified water | 1366.9 g |

### [[Overcoat layer 11A spray liquid]

| | |
|---|---|
| • Mannitol | 140.0 g |
| • Purified water | 860.0 g |

### <Production Example 12>

400 g of D-mannitol (trade name: NONPAREIL (registered trademark)-108) as core particles was introduced into a fluidized bed granulator (type: MP-01 (SPC)), and the charge air temperature of the fluidized bed granulator was adjusted to 80°C, the air supply rate to from 0.6 m³/h to 1 m³/h, and the liquid velocity to about 4 g/min.

The entire amount of an active pharmaceutical ingredient layer 12A spray liquid, the entire amount of an underlying layer 12A spray liquid, and the entire amount of an overlying layer 12A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 12A / intermediate layer 12A (underlying layer 12A / overlying layer 12A)] were obtained.

### [Active pharmaceutical ingredient layer 12A spray liquid]

| | |
|---|---|
| • Butylscopolamine bromide (active ingredient, antispasmodic agent) | 66.2 g |
| • Talc (water-insoluble particles) | 66.2 g |
| • Purified water | 88.3 g |

### [Underlying layer 12A spray liquid]

| | |
|---|---|
| • Hydroxypropyl cellulose (type SSL) 10 mass% aqueous solution (coating film component) | 31.7 g |
| • Mannitol | 21.1 g |
| • Talc (water-insoluble particles) | 5.3 g |
| • Purified water | 82.7 g |

### [Overlying layer 12A spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 193.2 g |
| • Mannitol | 19.3 g |
| • Talc (water-insoluble particles) | 7.7 g |
| • Purified water | 166.2 g |

400 g of the primary fine granules thus obtained were introduced into a fluidized bed granulator (type: MP-01 (SPC)), and the charge air temperature of the fluidized bed granulator was adjusted to 40°C, the air supply rate to from 0.6 m³/h to 1 m³/h, and the liquid velocity to about 4 g/min.

2009 g (defined amount) of a bitterness-masking layer 12A spray liquid and 363 g (defined amount) of an overcoat layer 12A spray liquid, all of which had compositions as described below and had been prepared in advance, were spray-coated successively in this order on the primary fine granules, and then drying was performed for 30 minutes by setting the charge air temperature of the fluidized bed granulator to 80°C. Thus, fine granules of Production Example 12 [layer configuration: core particle / active pharmaceutical ingredient layer 12A / intermediate layer 12A (underlying layer 12A / overlying layer 12A) / bitterness-masking layer 12A / overcoat layer 12A] were obtained.

### [Bitterness-masking layer 12A spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 627.8 g |
| • Aminoalkyl methacrylate copolymer RS dispersion liquid (water-insoluble polymer) (trade name: EUDRAGIT (registered trademark) RL30D, 30 mass% aqueous dispersion liquid, Evonik Industries AG) | 627.8 g |
| • Triethyl citrate | 37.7 g |
| • Talc | 188.3 g |
| • Purified water | 1531.9 g |

### [Overcoat layer 12A spray liquid]

| | |
|---|---|
| • Mannitol | 140.0 g |
| • Purified water | 860.0 g |

### [Evaluation of stability]

The contents of butylscopolamine bromide in the fine granules of Production Example 11 and Production Example 12 were quantitatively determined by HPLC (High Performance Liquid Chromatography). Furthermore, the fine granules of Production Example 11 and Production Example 12 were stored for 3 days at 80°C or for 7 days at 70°C, and the contents of butylscopolamine bromide after the storage were quantitatively determined by HPLC. Then, the proportions (%) of the content after storage with respect to the content before storage were calculated and used as an index for stability. The conditions for HPLC are presented below.

### - HPLC conditions -

Column: CAPCELLPAK C8 UG120 (Shiseido Co., Ltd.)
   (4.6 mm × 150 mm, pore size 5 µm)
Column temperature: 30°C
Eluent: Methanol/phosphate buffer solution (pH 3.6) = 68 / 37 (volume ratio)
Detection wavelength: 210 nm
Flow rate: 1 mL/min
Injection amount: 20 µL

### [Evaluation on bitterness concealing]

The fine granules of Production Example 11 and Production Example 12 were measured such that 10 mg of butylscopolamine bromide would be included in a dose, and the fine granules were inserted into the oral cavities of test subjects. The bitterness masking time was measured, and an evaluation on bitterness concealing was performed according to the following evaluation criteria.

If a sample is rated [A] or [B] of the following evaluation criteria, there is no problem in practical use.

### - Evaluation criteria -

A: The bitterness masking time is 60 seconds or more.
B: The bitterness masking time is 30 seconds or more and less than 60 seconds.
C: The bitterness masking time is less than 30 seconds.

The compositions and evaluation results for the fine granules of Production Example 11 and Production Example 12 are presented in Table 5. In Table 5, the unit "percent (%)" means "percent (%) by mass", and the symbol "-" implies that the relevant component has not been incorporated.

**[Table 5]**

| | | | Production Example 11 | Production Example 12 |
|---|---|---|---|---|
| Active pharmaceutical ingredient-containing core | Core particle | Mannitol | 30.2% | 31.0% |
| | Active pharmaceutical ingredient layer | Butylscopolamine bromide | 5.0% | 5.1% |
| | | Talc | - | 5.1% |
| | | Hydrous silicon dioxide | 5.0% | - |
| Intermediate layer | Underlying layer | Film thickness (µm) | 2 | 2 |
| | | Hydroxypropyl cellulose (HPC) | - | 0.2% |
| | | Mannitol | - | 1.6% |
| | | Talc | - | 0.4% |
| | | Hydrous silicon dioxide | 3.4% | - |
| | Overlying layer | Film thickness (µm) | 3 | 3 |
| | | Hydroxypropyl methylcellulose (HPMC) | - | 1.5% |
| | | Hydroxypropyl cellulose (HPC) | 1.5% | - |
| | | Mannitol | 1.5% | 1.5% |
| | | Talc | 0.6% | 0.6% |
| Bitterness-masking layer | | Aminoalkyl methacrylate polymer RS (EUDRAGIT RS30D) | 11.2% | 14.7% |
| | | Aminoalkyl methacrylate polymer RS (EUDRAGIT RL30D) | 11.2% | 14.7% |
| | | Ethyl acrylate/methyl methacrylate copolymer (EUDRAGIT NE30D) | 5.6% | - |
| | | Triethyl citrate | 2.2% | 2.9% |
| | | Talc | 16.7% | 14.7% |
| Overcoat layer | | Mannitol | 5.9% | 6.0% |
| Stability evaluation (80°C, for 3 days) | | | 95.6% | 91.3% |
| Stability evaluation (70°C, for 7 days) | | | 97.8% | 94.3% |
| Evaluation on bitterness concealing | | | A | A |

As shown in Table 5, in the fine granules of Production Example 11 and Production Example 12, the bitter taste attributed to butylscopolamine bromide was sufficiently concealed.

The fine granules of Production Example 11 that included hydrous silicon dioxide as water-insoluble particles in the active pharmaceutical ingredient layer and the underlying layer, exhibited excellent stability compared to the fine granules of Production Example 12 that included talc.

### [Evaluation of orally disintegrating tablet]

### <<Example 10>>

The fine granules of Production Example 9 and excipient components were mixed at the ratio of the following Formulation (3), and a tableting powder (mixed powder) was obtained.

A defined amount of the tableting powder (mixed powder) thus obtained was measured and was subjected to compression molding at a speed of rotation of 40 rpm and a tableting pressure of about 5 kN, using a rotary tableting machine (product name: HT-AP-SS, Hata Tekkosho, Ltd.) and using a pounder having an angular-cornered R face with 9 mmφ. Thus, an orally disintegrating tablet (tablet preparation) of Example 10 was obtained.

### [Formulation (3)]

| | |
|---|---|
| • Fine granules | 33.3% by mass |
| • Mannitol / corn starch granulated material | 34.8% by mass |
| • Ethyl cellulose | 4.0% by mass |
| • Crospovidone | 3.0% by mass |
| • Aspartame | 1.0% by mass |
| • Crystalline cellulose | 20.0% by mass |
| • Magnesium aluminometasilicate | 2.5% by mass |
| • Hydrous silicon dioxide | 1.0% by mass |
| • STRAWBERRY MICRON | 0.1% by mass |
| • Calcium stearate | 0.3% by mass |

### <<Example 11>>

The fine granules of Production Example 9 and excipient components were mixed at the ratio of the following Formulation (4), and a tableting powder (mixed powder) was obtained.

A defined amount of the tableting powder (mixed powder) thus obtained was measured and was subjected to compression molding at a speed of rotation of 20 rpm and a tableting pressure of about 6 kN, using a rotary tableting machine (product name: HT-AP-SS, Hata Tekkosho, Ltd.) and using a pounder having an angular-cornered R face with 9 mmφ. Thus, an orally disintegrating tablet (tablet preparation) of Example 11 was obtained.

### [Formulation (4)]

| | |
|---|---|
| • Fine granules | 33.3% by mass |
| • Mannitol / corn starch granulated material | 56.3% by mass |
| • Ethyl cellulose | 5.0% by mass |
| • Crospovidone | 3.0% by mass |
| • Sucralose | 1.0% by mass |
| • Hydrous silicon dioxide | 1.0% by mass |
| • STRAWBERRY MICRON | 0.1% by mass |
| • Calcium stearate | 0.3% by mass |

### <<Example 12>>

The fine granules of Production Example 10 and excipient components were mixed at the ratio of the following Formulation (5), and a tableting powder (mixed powder) was obtained.

A defined amount of the tableting powder (mixed powder) thus obtained was measured and was subjected to compression molding at a speed of rotation of 20 rpm and a tableting pressure of about 6 kN, using a rotary tableting machine (product name: HT-AP-SS, Hata Tekkosho, Ltd.) and using a pounder having an angular-cornered R face with 9 mmφ. Thus, an orally disintegrating tablet (tablet preparation) of Example 12 was obtained.

### [Formulation (5)]

| | |
|---|---|
| • Fine granules | 33.3% by mass |
| • Mannitol / corn starch granulated material | 55.8% by mass |
| • Ethyl cellulose | 5.0% by mass |
| • Crospovidone | 3.0% by mass |
| • Sucralose | 1.0% by mass |
| • Hydrous silicon dioxide | 1.0% by mass |
| • Sodium lauryl sulfate | 0.5% by mass |
| • STRAWBERRY MICRON | 0.1 % by mass |
| • Calcium stearate | 0.3% by mass |

### <<Example 13>>

The fine granules of Production Example 11 and excipient components were mixed at the ratio of the following Formulation (4), and a tableting powder (mixed powder) was obtained.

An orally disintegrating tablet (tablet preparation) of Example 13 was obtained in the same manner as in Example 11, using the tableting powder (mixed powder) thus obtained.

### [Evaluation on bitterness concealing]

The orally disintegrating tablets of Examples 10 to 13 thus obtained were inserted into the oral cavities of test subjects. In regard to the way of feeling the bitter taste attributed to butylscopolamine bromide 5 minutes after insertion, the bitter taste felt in the oral cavity as well as irritation at the pharynx were comprehensively evaluated, and the orally disintegrating tablets were rated into six groups, including from "0 (bitterness is not felt)" to "5 (bitterness is strongly felt)". When the rating is "2" or lower, there is no problem in practical use. The results are presented in Table 6. In Table 6, the unit "percent (%)" means "percent (%) by mass", and the symbol "-" means that the relevant component has not been incorporated.

**[Table 6]**

| | Example 10 | Example 11 | Example 12 | Example 13 |
|---|---|---|---|---|
| Fine granules (do not include sodium lauryl sulfate) | - | - | 33.3% | 33.3% |
| Fine granules (include sodium lauryl sulfate) | 33.3% | 33.3% | - | - |
| Mannitol/corn starch granulated material | 34.8% | 56.3% | 55.8% | 56.3% |
| Ethyl cellulose | 4.0% | 5.0% | 5.0% | 5.0% |
| Crospovidone | 3.0% | 3.0% | 3.0% | 3.0% |
| Sucralose | - | 1.0% | 1.0% | 1.0% |
| Aspartame | 1.0% | - | - | - |
| Crystalline cellulose | 20.0% | - | - | - |
| Magnesium aluminometasilicate | 2.5% | - | - | - |
| Hydrous silicon dioxide | 1.0% | 1.0% | 1.0% | 1.0% |
| Sodium lauryl sulfate | - | - | 0.5% | - |
| STRAWBERRY MICRON | 0.1% | 0.1% | 0.1% | 0.1% |
| Calcium stearate | 0.3% | 0.3% | 0.3% | 0.3% |
| Evaluation on bitterness concealing (bitterness remaining after 5 minutes) | 0 | 0 | 1 | 2 |

As shown in Table 6, the orally disintegrating tablets of Example 10 and Example 11 that included sodium lauryl sulfate (anionic substance having a pKa of 3.5 or lower) in the overcoat layer of the fine granules, and the orally disintegrating tablet of Example 12 that included sodium lauryl sulfate on the outside of the fine granules as an excipient component had an excellent effect of concealing the bitter taste attributed to butylscopolamine bromide, compared to the orally disintegrating tablet of Example 13 that did not include sodium lauryl sulfate. Furthermore, the orally disintegrating tablets of Examples 10 to 13 had hardness and disintegrability that were sufficient for practical use.

The orally disintegrating tablets of Example 10 and Example 11 that included sodium lauryl sulfate in the overcoat layer of the fine granules had an excellent effect of concealing the bitter taste attributed to butylscopolamine bromide, compared to the orally disintegrating tablet of Example 12 that included sodium lauryl sulfate on the outside of the fine granules as an excipient.

### [Production of fine granules]

### <Production Example 13>

400 g of D-mannitol (trade name: NONPAREIL (registered trademark)-108) as core particles was introduced into a fluidized bed granulator (type: MP-01 (SPC)), and the charge air temperature of the fluidized bed granulator was adjusted to 60°C, the air supply rate to from 0.6 m³/h to 1 m³/h, and the liquid velocity to about 4 g/min.

The entire amount of an active pharmaceutical ingredient layer 13A spray liquid, the entire amount of an underlying layer 13A spray liquid, and the entire amount of an overlying layer 13A spray liquid, all of which had compositions as described below and had been prepared in advance, were spray-coated in this order on the core particles, and then drying was performed for 30 minutes by setting the charge air temperature of the fluidized bed granulator to 80°C. Thus, primary fine granules [layer configuration: core particle / active pharmaceutical ingredient layer 13A / intermediate layer 13A (underlying layer 13A / overlying layer 13A)] were obtained.

### [Active pharmaceutical ingredient layer 13A spray liquid]

| | |
|---|---|
| • Butylscopolamine bromide (active ingredient, antispasmodic agent) | 107.5 g |
| • Hydroxypropyl cellulose (type SSL) 10 mass% aqueous solution (coating film component) | 161.3 g |
| • Talc (water-insoluble particles) | 26.9 g |
| • Purified water | 421.2 g |

### [Underlying layer 13A spray liquid]

| | |
|---|---|
| • Hydroxypropyl cellulose (type SSL) 10 mass% aqueous solution (coating film component) | 36.5 g |
| • Mannitol | 24.3 g |
| • Talc (water-insoluble particles) | 6.1 g |
| • Purified water | 95.2 g |

### [Overlying layer 13A spray liquid]

| | |
|---|---|
| • Hydroxypropyl methylcellulose (trade name: TC-5, product class: M, degree of substitution type: 2910, viscosity: 4.5 mPa·s, Shin-Etsu Chemical Co., Ltd.) | 223.6 g |
| • Mannitol | 22.4 g |
| • Talc (water-insoluble particles) | 8.9 g |
| • Purified water | 192.3 g |

12 g of the primary fine granules thus obtained were introduced into a micro fluidized bed granulating/coating apparatus (type: MICRO FLUID BED), and the charge air temperature of the apparatus was adjusted to 40°C, the air supply rate to from 28 L/min to 30 L/min, and the liquid velocity to about 0.3 g/min.

57.0 g (defined amount) of a bitterness-masking layer 13A spray liquid and 10.5 g (defined amount) of an overcoat layer 13A spray liquid, all of which had compositions as described below and had been prepared in advance, were spray-coated successively in this order on the primary fine granules, and then drying was performed for 30 minutes by setting the charge air temperature of the apparatus to 80°C. Thus, fine granules of Production Example 13 [layer configuration: core particle / active pharmaceutical ingredient layer 13A / intermediate layer 13A (underlying layer 13A / overlying layer 13A) / bitterness-masking layer 13A / overcoat layer 13A] were obtained.

### [Bitterness-masking layer 13A spray liquid]

| | |
|---|---|
| • Aminoalkyl methacrylate copolymer RS dispersion liquid (water-insoluble polymer) (trade name: EUDRAGIT (registered trademark) RS30D, 30 mass% aqueous dispersion liquid, Evonik Industries AG) | 11.9 g |
| • Aminoalkyl methacrylate copolymer RS dispersion liquid (water-insoluble polymer) (trade name: EUDRAGIT (registered trademark) RL30D, 30 mass% aqueous dispersion liquid, Evonik Industries AG) | 11.9 g |
| • Triethyl citrate | 0.71 g |
| • Talc | 3.6 g |
| • Purified water | 29.0 g |

### [Overcoat layer 13A spray liquid]

| | |
|---|---|
| • Mannitol | 140.0 g |
| • Purified water | 860.0 g |

### [Evaluation on bitterness concealing]

The fine granules of Production Example 13 were measured such that 10 mg of butylscopolamine bromide would be included in a dose, and the fine granules were inserted into the oral cavities of test subjects. The bitterness masking time was measured, and an evaluation on bitterness concealing was performed according to the following evaluation criteria.

If a sample is rated [A] or [B] of the following evaluation criteria, there is no problem in practical use.

### - Evaluation criteria -

A: The bitterness masking time is 60 seconds or more.
B: The bitterness masking time is 30 seconds or more and less than 60 seconds.
C: The bitterness masking time is less than 30 seconds.

The composition and evaluation results for the fine granules of Production Example 13 are presented in Table 7. In Table 7, the unit "percent (%)" means "percent (%) by mass", and the symbol "-" implies that the relevant component has not been incorporated.

**[Table 7]**

| | | | Production Example 13 |
|---|---|---|---|
| Active pharmaceutical ingredient-containing core | Core particle | Mannitol | 27.4% |
| | Active pharmaceutical ingredient layer | Butylscopolamine bromide | 7.4% |
| | | Hydroxypropyl cellulose (HPC) | 1.1% |
| | | Hydrous silicon dioxide | 1.8% |
| Intermediate layer | Underlying layer | Film thickness (µm) | 2 |
| | | Hydroxypropyl cellulose (HPC) | 0.3% |
| | | Mannitol | 1.7% |
| | | Talc | 0.4% |
| | | Hydrous silicon dioxide | - |
| | Overlying layer | Film thickness (µm) | 3 |
| | | Hydroxypropyl methylcellulose (HPMC) | 1.5% |
| | | Mannitol | 1.5% |
| | | Talc | 0.6% |
| Bitterness-masking layer | | Aminoalkyl methacrylate polymer RS (EUDRAGIT RS30D) | 15.6% |
| | | Aminoalkyl methacrylate polymer RS (EUDRAGIT RL30D) | 15.6% |
| | | Triethyl citrate | 3.1% |
| | | Talc | 15.6% |
| Overcoat layer | | Mannitol | 6.4% |
| Evaluation on bitterness concealing | | | A |

As shown in Table 7, in the fine granules of Production Example 13, the bitter taste attributed to butylscopolamine bromide was sufficiently concealed.

### [Evaluation of orally disintegrating tablet]

### <<Example 14>>

The fine granules of Production Example 13 and excipient components were mixed at the ratio of the following Formulation (6), and a tableting powder (mixed powder) was obtained.

A defined amount of the tableting powder (mixed powder) thus obtained was measured and was subjected to compression molding at a tableting pressure of about 3 kN, using a simple table molding machine (ENERPAC) and using a pounder having an SR face with 9 mmφ. Thus, an orally disintegrating tablet (tablet preparation) of Example 14 was obtained.

### [Formulation (6)]

| | |
|---|---|
| • Fine granules | 50.0% by mass |
| • Mannitol/corn starch granulated material | 35.5% by mass |
| • Ethyl cellulose | 5.1 % by mass |
| • Crospovidone | 3.0% by mass |
| • Aspartame | 1.0% by mass |
| • STRAWBERRY MICRON | 0.1% by mass |
| • Sodium stearyl fumarate | 0.8% by mass |
| • Sodium lauryl sulfate | 4.5% by mass |

### <<Example 15>>

The fine granules of Production Example 13 and excipient components were mixed at the ratio of the following Formulation (7), and a tableting powder (mixed powder) was obtained.

A defined amount of the tableting powder (mixed powder) thus obtained was measured and was subjected to compression molding at a tableting pressure of about 3 kN, using a simple table molding machine (ENERPAC) and using a pounder having an SR face with 9 mmφ. Thus, an orally disintegrating tablet (tablet preparation) of Example 15 was obtained.

### [Formulation (7)]

| | |
|---|---|
| • Fine granules | 50.0% by mass |
| • Mannitol/corn starch granulated material | 35.5% by mass |
| • Ethyl cellulose | 5.1% by mass |
| • Crospovidone | 3.0% by mass |
| • Aspartame | 1.0% by mass |
| • STRAWBERRY MICRON | 0.1% by mass |
| • Sodium stearyl fumarate | 0.8% by mass |
| • Sodium alginate | 4.5% by mass |

### <<Example 16>>

The fine granules of Production Example 13 and excipient components were mixed at the ratio of the following Formulation (8), and a tableting powder (mixed powder) was obtained.

A defined amount of the tableting powder (mixed powder) thus obtained was measured and was subjected to compression molding at a tableting pressure of about 3 kN, using a simple table molding machine (ENERPAC) and using a pounder having an SR face with 9 mmφ. Thus, an orally disintegrating tablet (tablet preparation) of Example 16 was obtained.

### [Formulation (8)]

| | |
|---|---|
| • Fine granules | 50.0% by mass |
| • Mannitol/corn starch granulated material | 40.0% by mass |
| • Ethyl cellulose | 5.1% by mass |
| • Crospovidone | 3.0% by mass |
| • Aspartame | 1.0% by mass |
| • STRAWBERRY MICRON | 0.1% by mass |
| • Sodium stearyl fumarate | 0.8% by mass |

### <Evaluation on bitterness concealing>

The orally disintegrating tablets of Examples 14 to 16 thus obtained were inserted into the oral cavities of test subjects. An evaluation was performed according to the following evaluation criteria, on whether the bitter taste attributed to butylscopolamine bromide was felt in the oral cavity 60 seconds after insertion. The results are presented in Table 8. In Table 8, the unit "percent (%)" means "percent (%) by mass", and the symbol "-" means that the relevant component has not been incorporated.

### - Evaluation criteria -

0: The bitter taste is not felt 60 seconds after insertion.
1: The bitter taste is slightly felt 60 seconds after insertion.

**[Table 8]**

| | Example 14 | Example 15 | Example 16 |
|---|---|---|---|
| Fine granules | 50.0% | 50.0% | 50.0% |
| Mannitol/corn starch granulated material | 35.5% | 35.5% | 40.0% |
| Ethyl cellulose | 5.1% | 5.1% | 5.1% |
| Crospovidone | 3.0% | 3.0% | 3.0% |
| Aspartame | 1.0% | 1.0% | 1.0% |
| STRAWBERRY MICRON | 0.1% | 0.1% | 0.1% |
| Sodium stearyl fumarate | 0.8% | 0.8% | 0.8% |
| Sodium lauryl sulfate | 4.5% | - | - |
| Sodium alginate | - | 4.5% | - |
| Evaluation on bitterness concealing | 0 | 0 | 1 |

As shown in Table 8, the orally disintegrating tablets of Example 14 and Example 15 that included sodium lauryl sulfate or sodium alginate (all are anionic substances having a pKa of 3.5 or lower) on the outside of the fine granules as an excipient component, had an excellent effect of concealing the bitter taste attributed to butylscopolamine bromide, compared to the orally disintegrating tablet of Example 16 that did not include an anionic substance having a pKa of 3.5 or lower.

## Claims

1. An orally disintegrating tablet comprising:
fine granules, each fine granule having, at its center, an active pharmaceutical ingredient-containing core that comprises butylscopolamine bromide and water-insoluble particles, and having an intermediate layer that comprises water-insoluble particles and coats the active pharmaceutical ingredient-containing core, and a bitterness-masking layer that comprises talc and at least one water-insoluble polymer, in sequence from the active pharmaceutical ingredient-containing core side; and
an excipient component positioned on the outside of the fine granules.

2. The orally disintegrating tablet according to claim 1, wherein the fine granule further has an overcoat layer comprising an anionic substance having a pKa of 3.5 or lower, as a layer positioned on an outer side of the bitterness-masking layer.

3. The orally disintegrating tablet according to claim 1 or 2, wherein a thickness of the intermediate layer is 10 µm or less.

4. The orally disintegrating tablet according to any one of claims 1 to 3, wherein an average particle size of the fine granules is from 100 µm to 500 µm.

5. The orally disintegrating tablet according to any one of claims 1 to 4, wherein the intermediate layer has an underlying layer and an overlying layer in sequence from the active pharmaceutical ingredient-containing core side.

6. The orally disintegrating tablet according to claim 5, wherein the underlying layer comprises water-insoluble particles, or water-insoluble particles and a coating film component, and a ratio of a content of the water-insoluble particles to a content of the coating film component in the underlying layer is from 1.0:0.0 to 1.0:1.0 on a mass basis.

7. The orally disintegrating tablet according to any one of claims 1 to 6, wherein the active pharmaceutical ingredient-containing core has a core particle at its center, and has an active pharmaceutical ingredient layer comprising butylscopolamine bromide and water-insoluble particles on an outside of the core particle.

8. A method for producing an orally disintegrating tablet, the method comprising: obtaining fine granules by a production process comprising:
(A) spraying, onto a core particle that is configured to serve as a center of an active pharmaceutical ingredient-containing core, a spray liquid that comprises butylscopolamine bromide and water-insoluble particles and that is for forming an active pharmaceutical ingredient layer, so as to coat the core particle with the active pharmaceutical ingredient layer;
(B) spraying, onto the active pharmaceutical ingredient-containing core in which the core particle has been coated with the active pharmaceutical ingredient layer, a spray liquid that comprises water-insoluble particles and that is for forming an intermediate layer, so as to coat the active pharmaceutical ingredient-containing core with the intermediate layer; and
(C) spraying, onto the active pharmaceutical ingredient-containing core that has been coated with at least the intermediate layer, a spray liquid that comprises talc and at least one water-insoluble polymer and that is for forming a bitterness-masking layer, so as to coat the active pharmaceutical ingredient-containing core with the bitterness-masking layer; and
mixing the obtained fine granules with an excipient component.

9. The method for producing an orally disintegrating tablet according to claim 8, wherein the (B) comprises:
(B1) spraying, onto the active pharmaceutical ingredient-containing core, a spray liquid that comprises water-insoluble particles and that is for forming an underlying layer, so as to coat the active pharmaceutical ingredient-containing core with the underlying layer; and
(B2) spraying, onto the active pharmaceutical ingredient-containing core that has been coated with at least the underlying layer, a spray liquid that comprises water-insoluble particles and that is for forming an overlying layer, so as to coat the active pharmaceutical ingredient-containing core with the overlying layer.

10. The method for producing an orally disintegrating tablet according to claim 9 wherein a content of the water-insoluble particles in the spray liquid for forming an underlying layer is from 1.0% by mass to 50.0% by mass with respect to a total amount of the spray liquid for forming an underlying layer.

11. The orally disintegrating tablet according to any one of claims 1 to 7 or the method for producing an orally disintegrating tablet according to any one of claims 8 to 10, wherein the excipient component on the outside of the fine granules or the excipient component comprises an anionic substance having a pKa of 3.5 or lower.

12. The orally disintegrating tablet according to any one of claims 1 to 7 or 11, wherein the anionic substance having a pKa of 3.5 or lower is sodium lauryl sulfate.

13. The orally disintegrating tablet according to any one of claims 1 to 7, 11 or 12 or the method for producing an orally disintegrating tablet according to any one of claim 8 to 11, wherein the water-insoluble polymer comprises at least one selected from the group consisting of an aminoalkyl methacrylate copolymer RS and an ethyl acrylate/methyl methacrylate copolymer.

14. The orally disintegrating tablet according to any one of claims 1 to 7 or 11 to 13 or the method for producing an orally disintegrating tablet according to any one of claims 8 to 11 or 13, wherein the bitterness-masking layer or the spray liquid for forming a bitterness-masking layer comprises triethyl citrate at a proportion of from 5% by mass to 15% by mass with respect to a total solid content of the water-insoluble polymer.

15. The orally disintegrating tablet according to any one of claims 1 to 7 or 11 to 14 or the method for producing an orally disintegrating tablet according to any one of claim 8 to 11, 13 or 14, wherein the water-insoluble particles included in the active pharmaceutical ingredient-containing core and the intermediate layer or the water-insoluble particles included in the spray liquid for forming an active pharmaceutical ingredient layer and the spray liquid for forming an intermediate layer are formed of at least one selected from the group consisting of hydrous silicon dioxide, light anhydrous silicic acid, sodium stearyl fumarate, magnesium stearate, and talc.

## Patentansprüche

1. Im Mund zerfallende Tablette, umfassend:
ein Feingranulat, wobei jedes Feingranulatkorn in seiner Mitte einen pharmazeutischen wirkstoffhaltigen Kern, der Butylscopolaminbromid und wasserunlösliche Partikel umfasst, aufweist und eine Zwischenschicht, die wasserunlösliche Partikel umfasst und den pharmazeutischen wirkstoffhaltigen Kern bedeckt, aufweist und eine bitterkeitsmaskierende Schicht, die Talk und wenigstens ein wasserunlösliches Polymer umfasst, in der Reihenfolge ausgehend von der Seite des pharmazeutischen wirkstoffhaltigen Kerns aufweist; und
eine auf der Außenseite des Feingranulatkorns angeordnete Hilfsstoffkomponente.

2. Im Mund zerfallende Tablette gemäß Anspruch 1, wobei das Feingranulatkorn weiterhin eine Überzugschicht, umfassend einen anionischen Stoff mit einem pKa-Wert von 3,5 oder weniger, als eine auf einer äußeren Seite der bitterkeitsmaskierenden Schicht angeordnete Schicht aufweist.

3. Im Mund zerfallende Tablette gemäß Anspruch 1 oder 2, wobei die Dicke der Zwischenschicht 10 µm oder weniger beträgt.

4. Im Mund zerfallende Tablette gemäß einem der Ansprüche 1 bis 3, wobei die durchschnittliche Partikelgröße des Feingranulats von 100 µm bis 500 µm beträgt.

5. Im Mund zerfallende Tablette gemäß einem der Ansprüche 1 bis 4, wobei die Zwischenschicht eine Unterschicht und eine Überschicht in der Reihenfolge ausgehend von der Seite des pharmazeutischen wirkstoffhaltigen Kerns aufweist.

6. Im Mund zerfallende Tablette gemäß Anspruch 5, wobei die Unterschicht wasserunlösliche Partikel oder wasserunlösliche Partikel und eine Beschichtungsfilmkomponente umfasst und das Verhältnis des Gehalts der wasserunlöslichen Partikel zum Gehalt der Beschichtungsfilmkomponente in der Unterschicht von 1,0:0,0 bis 1,0:1,0 auf Massenbasis beträgt.

7. Im Mund zerfallende Tablette gemäß einem der Ansprüche 1 bis 6, wobei der pharmazeutische werkstoffhaltige Kern ein Kernpartikel in seiner Mitte aufweist und eine pharmazeutische wirkstoffhaltige Schicht, die Butylscopolaminbromid und wasserunlösliche Partikel auf einer Außenseite des Kernpartikels umfasst, aufweist.

8. Verfahren zum Herstellen einer im Mund zerfallenden Tablette, wobei das Verfahren umfasst:
Erhalten eines Feingranulats durch ein Herstellungsverfahren, umfassend:
(A) Besprühen eines Kernpartikels, derart ausgestaltet, dass es als ein Zentrum eines pharmazeutischen wirkstoffhaltigen Kerns dienen kann, mit einer Sprühflüssigkeit, die Butylscopolaminbromid und wasserunlösliche Partikel umfasst und zur Bildung einer pharmazeutischen wirkstoffhaltigen Schicht vorgesehen ist, so dass das Kernpartikel mit der pharmazeutischen wirkstoffhaltigen Schicht beschichtet wird;
(B) Besprühen des pharmazeutischen wirkstoffhaltigen Kerns, in dem das Kernpartikel mit der pharmazeutischen wirkstoffhaltigen Schicht beschichtet worden ist, mit einer Sprühflüssigkeit, die wasserunlösliche Partikel umfasst und zur Bildung einer Zwischenschicht vorgesehen ist, so dass der pharmazeutische werkstoffhaltige Kern mit der Zwischenschicht beschichtet wird; und
(C) Besprühen des pharmazeutischen wirkstoffhaltigen Kerns, der wenigstens mit der Zwischenschicht beschichtet worden ist, mit einer Sprühflüssigkeit, die Talk und wenigstens ein wasserunlösliches Polymer umfasst und zur Bildung einer bitterkeitsmaskierenden Schicht vorgesehen ist, so dass der pharmazeutische werkstoffhaltige Kern mit der bitterkeitsmaskierenden Schicht beschichtet wird; und
Vermischen des erhaltenen Feingranulats mit einer Hilfsstoffkomponente.

9. Verfahren zum Herstellen einer im Mund zerfallenden Tablette gemäß Anspruch 8, wobei (B) umfasst:
(B1) Besprühen des pharmazeutischen wirkstoffhaltigen Kerns mit einer Sprühflüssigkeit, die wasserunlösliche Partikel umfasst und zur Bildung einer Unterschicht vorgesehen ist, so dass der pharmazeutische werkstoffhaltige Kern mit der Unterschicht beschichtet wird; und
(B2) Besprühen des pharmazeutischen wirkstoffhaltigen Kerns, der wenigstens mit der Unterschicht beschichtet worden ist, mit einer Sprühflüssigkeit, die wasserunlösliche Partikel umfasst und zur Bildung einer Überschicht vorgesehen ist, so dass der pharmazeutische Werkstoffkern mit der Überschicht beschichtet wird.

10. Verfahren zum Herstellen einer im Mund zerfallenden Tablette gemäß Anspruch 9, wobei der Anteil der wasserunlöslichen Partikel in der Sprühflüssigkeit zur Bildung einer Unterschicht von 1,0 Masse-% bis 50,0 Masse-% in Bezug auf die Gesamtmenge der Sprühflüssigkeit zur Bildung einer Unterschicht beträgt.

11. Im Mund zerfallende Tablette gemäß einem der Ansprüche 1 bis 7 oder Verfahren zum Bilden einer im Mund zerfallenden Tablette gemäß einem der Ansprüche 8 bis 10, wobei die Hilfsstoffkomponente auf der Außenseite des Feingranulats oder die Hilfsstoffkomponente einen anionischen Stoff mit einem pKa-Wert von 3,5 oder weniger umfasst.

12. Im Mund zerfallende Tablette gemäß einem der Ansprüche 1 bis 7 oder 11, wobei der anionische Stoff mit einem pKa-Wert von 3,5 oder weniger Natriumlaurylsulfat ist.

13. Im Mund zerfallende Tablette gemäß einem der Ansprüche 1 bis 7, 11 oder 12 oder Verfahren zum Herstellen einer im Mund zerfallenden Tablette gemäß einem der Ansprüche 8 bis 11, wobei das wasserunlösliche Polymer wenigstens ein Polymer, ausgewählt aus Gruppe, bestehend aus einem Aminoalkylmethacrylat-Copolymer RS und einem Ethylacrylat/Methylmethacrylat-Copolymer, umfasst.

14. Im Mund zerfallende Tablette gemäß einem der Ansprüche 1 bis 7 oder 11 bis 13 oder Verfahren zum Herstellen einer im Mund zerfallenden Tablette gemäß einem der Ansprüche 8 bis 11 oder 13, wobei die bitterkeitsmaskierende Schicht oder die Sprühflüssigkeit zur Bildung einer bitterkeitsmaskierenden Schicht Triethylcitrat in einem Anteil von 5 Masse-% bis 15 Masse-% in Bezug auf den Gesamtfeststoffgehalt des wasserunlöslichen Polymers umfasst.

15. Im Mund zerfallende Tablette gemäß einem der Ansprüche 1 bis 7 oder 11 bis 14 oder Verfahren zum Herstellen einer im Mund zerfallenden Tablette gemäß einem der Ansprüche 8 bis 11, 13 oder 14, wobei die im pharmazeutischen wirkstoffhaltigen Kern oder in der Zwischenschicht enthaltenen wasserunlöslichen Partikel oder die in der Sprühflüssigkeit zur Bildung eines pharmazeutischen wirkstoffhaltigen Kerns und der Sprühflüssigkeit zur Bildung einer Zwischenschicht enthaltenen wasserunlöslichen Partikel aus wenigstens einem Stoff, ausgewählt aus der Gruppe, bestehend aus wasserhaltigem Siliciumdioxid, leichter wasserfreier Kieselsäure, Natriumstearylfumarat, Magnesiumstearat und Talk, gebildet sind.

## Revendications

1. Comprimé à désintégration intrabuccale comprenant :
des granules fins, chaque granule fin ayant, en son centre, un coeur contenant un principe pharmaceutique actif qui comprend du bromure de butylscopolamine et des particules insolubles dans l'eau, et qui est pourvu d'une couche intermédiaire qui comprend des particules insolubles dans l'eau et revêt le coeur contenant le principe pharmaceutique actif, et d'une couche masquant l'amertume qui comprend du talc et au moins un polymère insoluble dans l'eau, dans cet ordre à partir du côté coeur contenant un principe pharmaceutique actif ; et
un composant excipient positionné sur l'extérieur des granules fins.

2. Comprimé à désintégration intrabuccale selon la revendication 1, dans lequel le granule fin a en outre une couche de surenrobage comprenant une substance anionique ayant une pKa de 3,5 ou moins, en tant que couche positionnée sur un côté extérieur de la couche masquant l'amertume.

3. Comprimé à désintégration intrabuccale selon la revendication 1 ou 2, dans lequel une épaisseur de la couche intermédiaire est de 10 µm ou moins.

4. Comprimé à désintégration intrabuccale selon l'une quelconque des revendications 1 à 3, dans lequel une taille moyenne de particule des granules fins est de 100 µm à 500 µm.

5. Comprimé à désintégration intrabuccale selon l'une quelconque des revendications 1 à 4, dans lequel la couche intermédiaire a une couche sous-jacente et une couche sus-jacente dans cet ordre à partir du côté coeur contenant un principe pharmaceutique actif.

6. Comprimé à désintégration intrabuccale selon la revendication 5, dans lequel la couche sous-jacente comprend des particules insolubles dans l'eau, ou des particules insolubles dans l'eau et un composant film de revêtement, et un rapport d'une teneur en particules insolubles dans l'eau à une teneur en composant film de revêtement dans la couche sous-jacente est de 1,0:0,0 à 1,0:1,0 sur une base en masse.

7. Comprimé à désintégration intrabuccale selon l'une quelconque des revendications 1 à 6, dans lequel le coeur contenant un principe pharmaceutique actif a une particule centrale en son centre, et a une couche de principe pharmaceutique actif comprenant du bromure de butylscopolamine et des particules insolubles dans l'eau sur un extérieur de la particule centrale.

8. Procédé de production d'un comprimé à désintégration intrabuccale, le procédé comprenant :
l'obtention de fins granules par un procédé de production comprenant :
(A) la pulvérisation, sur une particule centrale qui est configurée pour servir de centre d'un coeur contenant un principe pharmaceutique actif, d'un liquide de pulvérisation qui comprend du bromure de butylscopolamine et des particules insolubles dans l'eau et qui est destiné à former une couche de principe pharmaceutique actif, de manière à revêtir la particule centrale de la couche de principe pharmaceutique actif ;
(B) la pulvérisation, sur le coeur contenant un principe pharmaceutique actif dans lequel la particule centrale a été revêtue de la couche de principe pharmaceutique actif, d'un liquide de pulvérisation qui comprend des particules insolubles dans l'eau et qui est destiné à former une couche intermédiaire, de manière à revêtir le coeur contenant un principe pharmaceutique actif avec la couche intermédiaire ; et
(C) la pulvérisation, sur le coeur contenant le principe pharmaceutique actif qui a été revêtu d'au moins la couche intermédiaire, d'un liquide de pulvérisation qui comprend du talc et au moins un polymère insoluble dans l'eau qui est destiné à former une couche masquant l'amertume, de manière à revêtir le coeur contenant un principe pharmaceutique actif de la couche masquant l'amertume ; et
le mélange des granules fins obtenus avec un composant excipient.

9. Procédé de production d'un comprimé à désintégration intrabuccale selon la revendication 8, dans lequel le (B) comprend :
(B1) la pulvérisation, sur le coeur contenant un principe pharmaceutique actif, d'un liquide de pulvérisation qui comprend des particules insolubles dans l'eau et qui est destiné à former une couche sous-jacente, de manière à revêtir le coeur contenant un principe pharmaceutique actif de la couche sous-jacente ; et
(B2) la pulvérisation, sur le coeur contenant le principe pharmaceutique actif qui a été revêtu d'au moins la couche sous-jacente, d'un liquide de pulvérisation qui comprend des particules insolubles dans l'eau et qui est destiné à former une couche sus-jacente, de manière à revêtir le coeur contenant un principe pharmaceutique actif de la couche sus-jacente.

10. Procédé de production d'un comprimé à désintégration intrabuccale selon la revendication 9 dans lequel une teneur en les particules insolubles dans l'eau dans le liquide de pulvérisation destiné à former une couche sous-jacente est de 1,0 % en masse à 50,0 % en masse relativement à une quantité totale du liquide de pulvérisation destiné à former une couche sous-jacente.

11. Comprimé à désintégration intrabuccale selon l'une quelconque des revendications 1 à 7 ou procédé de production d'un comprimé à désintégration intrabuccale selon l'une quelconque des revendications 8 à 10, dans lequel le composant excipient sur l'extérieur des granules fins ou le composant excipient comprend une substance anionique ayant une pKa de 3,5 ou moins.

12. Comprimé à désintégration intrabuccale selon l'une quelconque des revendications 1 à 7 ou 11, dans lequel la substance anionique ayant une pKa de 3,5 ou moins est le laurylsulfate de sodium.

13. Comprimé à désintégration intrabuccale selon l'une quelconque des revendications 1 à 7, 11 ou 12 ou procédé de production d'un comprimé à désintégration intrabuccale selon l'une quelconque des revendications 8 à 11, dans lequel le polymère insoluble dans l'eau comprend au moins un sélectionné dans le groupe constitué d'un copolymère de méthacrylate d'aminoalkyle RS et d'un copolymère acrylate d'éthyle/méthacrylate de méthyle.

14. Comprimé à désintégration intrabuccale selon l'une quelconque des revendications 1 à 7 ou 11 à 13 ou procédé de production d'un comprimé à désintégration intrabuccale selon l'une quelconque des revendications 8 à 11 ou 13, dans lequel la couche masquant l'amertume ou le liquide de pulvérisation destiné à former une couche masquant l'amertume comprend du citrate de triéthyle en une proportion de 5 % en masse à 15 % en masse relativement à une teneur totale en matières solides du polymère insoluble dans l'eau.

15. Comprimé à désintégration intrabuccale selon l'une quelconque des revendications 1 à 7 ou 11 à 14 ou procédé de production d'un comprimé à désintégration intrabuccale selon l'une quelconque des revendications 8 à 11, 13 ou 14, dans lequel les particules insolubles dans l'eau incluses dans le coeur contenant un principe pharmaceutique actif et la couche intermédiaire ou les particules insolubles dans l'eau incluses dans le liquide de pulvérisation destiné à former une couche de principe pharmaceutique actif et le liquide de pulvérisation destiné à former une couche intermédiaire sont formées d'au moins un sélectionné dans le groupe constitué du dioxyde de silicium hydreux, de l'acide silicique anhydre léger, du stéaryl fumarate de sodium, du stéarate de magnésium, et du talc.
